# EUROPEAN PATENT APPLICATION

(11) **EP 2 946 779 A1**
(43) Date of publication of application: **25.11.2015**
(21) Application number: 15000559.3
(22) Date of filing: 21.02.2008
(51) Int. Cl.: A61K 31/401, A61P 17/02, A61P 17/04, A61P 17/16, A61P 25/16, A61P 25/28, A61P 29/00

(54) **COSMETIC AND PHARMACEUTICAL APPLICATIONS OF N-ACETYLHYDROXYPROLINE**

(30) Priority: 22.02.2007 US 902491 P; 22.02.2007 US 902508 P; 22.02.2007 US 902509 P; 22.02.2007 US 902510 P
(62) Divisional of application: 08715906.7
(71) Applicant: Beiersdorf AG, 20253 Hamburg (DE)
(72) Inventor: Blatt, Thomas, DE - 22880 Wedel (DE); Mummert, Christopher, DE - 29553 Bienenbüttel (DE); Muhr, Gesa-Meike, DE - 20251 Hamburg (DE); Küper, Thomas, DE - 48734 Reken (DE); Wöhrmann, Yvonne, DE - 22851 Norderstedt (DE); Jaspers, Sören, DE - 22869 Schenefeld (DE); Kruse, Inge, DE - 20146 Hamburg (DE); Raschke, Thomas, DE - 25421 Pinneberg (DE); Schönrock, Uwe, DE - 23866 Nahe (DE); Koop, Urte, DE - 20149 Hamburg (DE)

(57) **Abstract**

Disclosed are cosmetic and pharmaceutical applications of N-acetylhydroxyproline and in particular, the use of N-acetylhydroxyproline for inhibiting the glycation of proteins in a living organism.

## Description

### CROSS-REFERENCE TO RELATED APPLICATIONS

The present application claims priority of U.S. Provisional Application Nos. 60/902,491, 60/902,508, 60/902,509 and 60/902,510, all filed February 22, 2007, the entire disclosures whereof are incorporated by reference herein.

### BACKGROUND OF THE INVENTION

The present invention relates generally to cosmetic and pharmaceutical applications of N-acetylhydroxyproline (hereafter sometimes abbreviated as NAHP). In particular, the present invention relates to the use of N-acetylhydroxyproline for inhibiting the glycation of proteins in a living organism, in particular, the human body.

The formation of advanced glycation endproducts (AGEs) is the result of the reaction between reducing sugars and amino groups of proteins based on the Maillard reaction. This reaction is termed glycation or non-enzymatic glycosylation¹. AGEs constitute of a heterogeneous group of structures while N^{ε}-(carboxymethyl)lysine (CML) adducts are the most prevalent AGEs *present in vivo*^{1,2}. CML along with other AGEs accumulates during normal ageing³⁻⁸ leading to stiffening and a lack of elasticity in connective tissues and vessel walls⁹. This accumulation is enhanced in diabetic patients⁹ as well as in human lung cancer tissues¹⁰, here attributed to a high glycolytic rate of tumours in general. Furthermore, AGEs are considered to be markers of various additional diseases, such as atheriosclerosis, renal failure and Alzheimer's disease^{3,8,11}.

In the current view the longevity of proteins is the major catalyst for the glycation reaction due to the extended time required for glycation under physiological conditions. In skin, AGE deposits have been observed in long-lived proteins such as fibronectin, laminin, collagen¹² and elastic¹. Additionally, actinic elastosis tissues contain AGE accumulations based on the glycation of elastic fibres that play a significant role in photoageing¹.

AGEs were initially thought to arise mainly from the reaction between extracellular proteins and glucose. However, recent studies report that the intracellular AGE formation from glucose-derived dicarbonyl precursors is much higher compared to the extracellular formation^{13,14}. These dicarbonyl precursors mainly comprise glyoxal, methylglyoxal and 3-deoxyglucosone¹⁵ which are substrates for reductases¹⁶. Glyoxal and methylglyoxal are detoxified by the glyoxalase system¹⁷. Intracellular AGEs induce oxidative stress, activate NF-κB and heme oxygenase, produce lipid peroxidation products, and cross-link proteins¹⁸. Until now only a few examples are known in which AGEs were degraded intracellularly^{19,20}, others report that cells store AGEs in lysosomes or phagosomes²¹ due to the persistent character of the glycated proteins.

The present inventors have identified vimentin as the major target for CML modification in human dermal fibroblasts. There is strong evidence that the biological impact of this modification is related to the loss of contractile capacity of fibroblasts caused by the structural breakdown of the intermediate filament system finally accelerating the process of ageing.

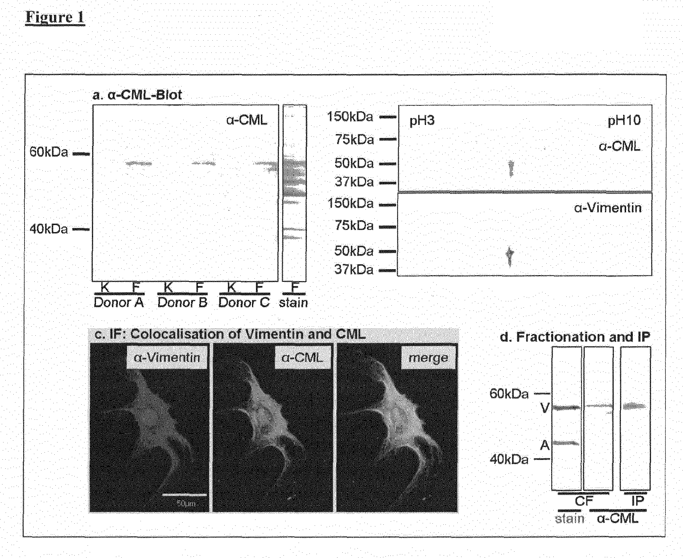

In particular, lysates of primary human fibroblasts and keratinocytes isolated from healthy donors were analysed by SDS page and immunoblotting for CML epitopes. Fibroblast lysates exhibited one prominent CML signal in the lanes of each human donor. This single band was not detected in the protein pool of the keratinocytes (Fig.1a). This result was confirmed using two-dimensional electrophoresis (Fig.1b, top) leading to one distinct spot positive for CML. The CML signal was analysed using nanoLC-ESI-MS/MS and identified as vimentin with a sequence coverage of 47%. Due to that, immunoblotting for vimentin (Fig.1b, bottom) was performed resulting in a strong positional match of the two signals representing vimentin and CML. To test whether the vimentin signal and the CML signal also overlap also in the cell immunofluorescence studies were conducted. Analysis by immunofluorescence microscopy (Fig.1c) showed a strong colocalisation of the two fluorescence signals. This colocalisation is present especially in the perinuclear region and to a lesser extent, in the distal areas of the cytosol where non-modified vimentin is located. In further fractionation studies vimentin and actin were identified as components of the fibroblast cytoskeletal fraction (Fig.1d, CF). In this fraction only vimentin exhibited a detectable CML-signal (Fig.1d, middle lane).

Surprisingly, the protein actin did not afford a detectable CML-signal in the fractionation studies, even though it was present in high amounts in the cytoskeletal fraction according to the silver-staining pattern. This clearly indicates a preferential CML-modification of vimentin. To further validate that the signal for colocalisation of CML and vimentin is not due to an accidental colocalisation with another cytoskeletal protein, immunoprecipitation studies were conducted. As demonstrated in Fig.1d (IP) the precipitated vimentin showed a strong CML-signal.

The current view of the non-enzymatic glycosylation reactions favours the protein half-life as a major factor which contributes to the formation of CML- and other derivates. In other words, a slow turnover of a protein is considered to be the most prominent reason for a high glycation rate of proteins^{5,8}. Therefore, the turnover of vimentin in comparison to other long-lived proteins such as actin and GAPDH in human skin fibroblasts was tested.

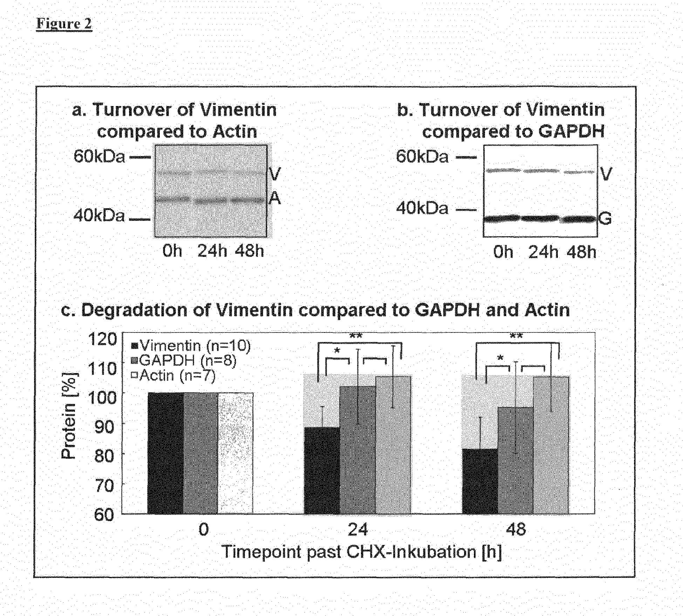

The turnover of vimentin was measured by using cycloheximide as an inhibitor of the protein *de novo* synthesis. The degradation of vimentin after cycloheximide addition to cultures of primary human fibroblasts was compared to the degradation of actin and GAPDH. Degradation of vimentin is faster compared to that of actin (Figures 2a,c) and GAPDH (Figures 2b,c). Actin and GAPDH are abundant proteins in skin fibroblasts (as is vimentin)²², but were not detected in the CML immunoblots in Figure 1. This finding is at variance with the current view which favors a long half-life as the major risk factor for the non-enzymatic glycosylation reaction. At least in the case of vimentin there are other prerequisites for the exceptionally high CML-modification. Notably, the intracellular localisation might be an important risk factor for such a modification. However, this seems to be unlikely in the case of vimentin taking into account that the distribution of vimentin and actin, both cytoskeletal proteins, is similar. Another factor to be considered are prerequisites regarding the structure of the protein molecule which might favour such modifications. To investigate this aspect in detail it was examined whether the modification of vimentin is based on intrinsic structural properties.

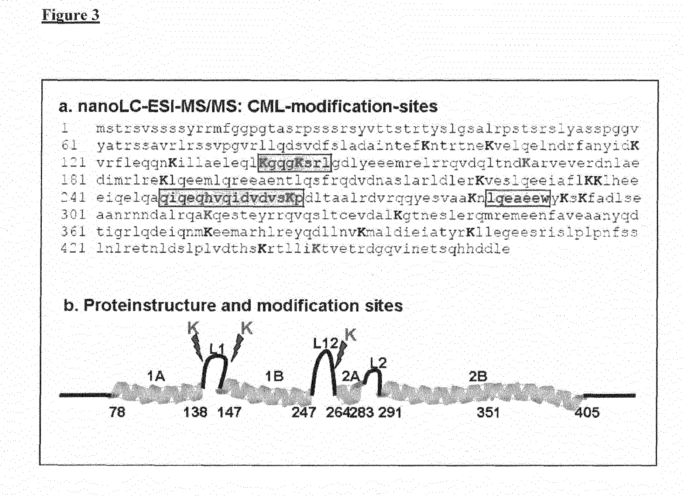

The present inventors have determined the exact locations of the modification sites of vimentin by nanoLC-ESI-MS/MS. Specifically, vimentin was isolated directly from fibroblasts by cell fractionation and the molecule was analyzed for modified lysine units. This makes it possible to detect modifications generated intracellularly to identify the modification sites which are present *in vivo.* This is an advantage over most of the presently available studies based *on in vitro* modified proteins²³. Figure 3a summarises the results of several nanoLC-ESI-MS/MS analyses. Lysine units found to be CML-modified to some extent are marked in red color. Remarkably, all lysine units located in the linker regions of the vimentin molecule²⁴ are modified by CML to some extent. In the folded protein domains the degree of modification is less prominent. According to Strelkov et al.²⁴ the linker regions are exposed to the surrounding area and seem to be, therefore, susceptible for CML-formation (Figure 3b).

In the case of long-lived proteins such as connective tissue collagen, glomerular basement membrane, and nerve proteins, roughly 1-3% of lysine residues are modified by glucose in normal individuals.⁹

. The present inventors have found that vimentin in fibroblasts is the preferential intracellular protein CML-modified in human skin. This high degree of modification seems to be primarily based on structural properties of the vimentin molecule and not on half-life or intracellular localisation.

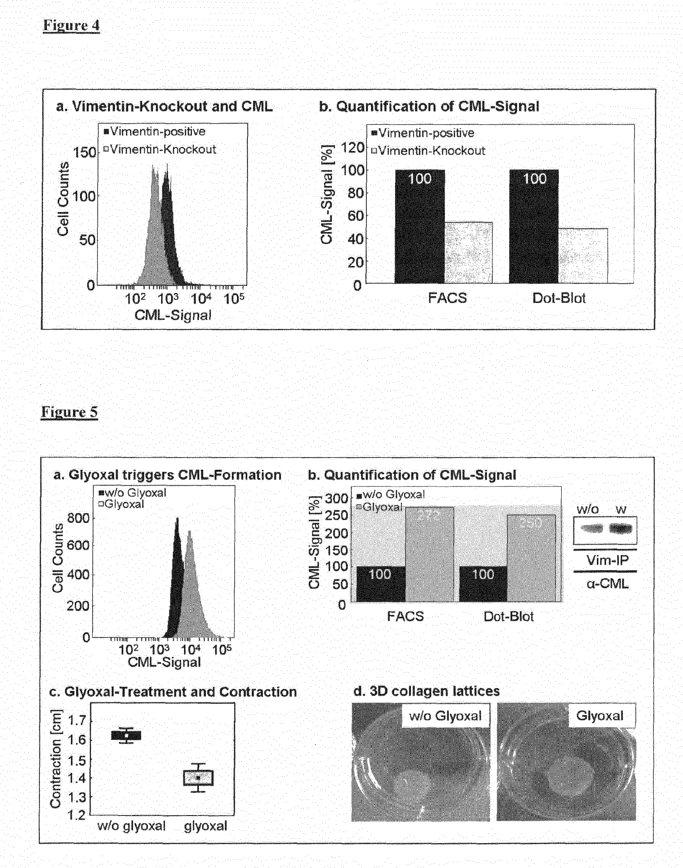

In order to test whether vimentin deletion results in lower CML-modifications a murine fibroblast cell line and a vimentin (-/-) sub-clone of these cells were employed and levels of CML-modifications were compared. Both FACS analysis (Figures 4a,b) and immunoblot analysis (Figure 4b, right), displayed a strong reduction in mean CML signal in vimentin-deficient fibroblasts compared to vimentin-positive fibroblasts. The mean CML signal in the knockout fibroblasts is 54 % of the value of the mean signal of vimentin positive fibroblasts. Dot blots showed similar results, indicating a reduction of 48% in CML level between vimentin positive and vimentin negative fibroblasts. These data confirm the high importance of vimentin as major target for CML-modification in human skin.

Eckes et al.²⁵ reported earlier on a reduced contractile capacity of vimentin-deficient fibroblasts compared to vimentin-positive fibroblasts, accompanied by a diminished stiffness of the knockout fibroblasts.

To test the effect of CML-modifications on vimentin function cells were treated with glyoxal to induce CML-vimentin modifications in fibroblasts. Primary human fibroblasts were incubated with 200 µM glyoxal for 7 days to enhance CML formation^{18,20,28}. This incubation resulted in a massive increase of CML as measured by FACS (Figures 5a,b) and immunoblot analysis (Figure 5b). Immunoprecipitation studies (Figure 5b, right) also showed a strong enhancement of CML-modification in vimentin after glyoxal treatment. The higher rate of CML-vimentin formation leads to a predominant modification of vimentin in the linker region of the molecule.

Further, normal fibroblasts were compared with fibroblasts with a higher CML-vimentin modification with respect to contractile capacities. The contractile capacity was determined using 3D free-floating collagen lattices^{29,30}. Any increase in CML-vimentin leads to a significantly (p=0.00056) diminished contractile capacity of the fibroblasts (Figure 5c,d) when seeded into a free-floating 3D collagen gel^{29,30}. The difference in this capacity is displayed by a collagen gel that is less contracted (Figure 5d, right) compared to the contracted gel containing the untreated control fibroblasts (Figure 5d, left).

It was surprisingly found that vimentin is preferentially modified by CML and modified vimentin loses its functionality while on the other hand, the modified enzyme is not degraded as shown by turnover studies. This - in consequence - raises the question as to what happens to the CML-modified protein. One of the possibilities is the sequestration of such proteins into aggregates as shown before^{31,32}.

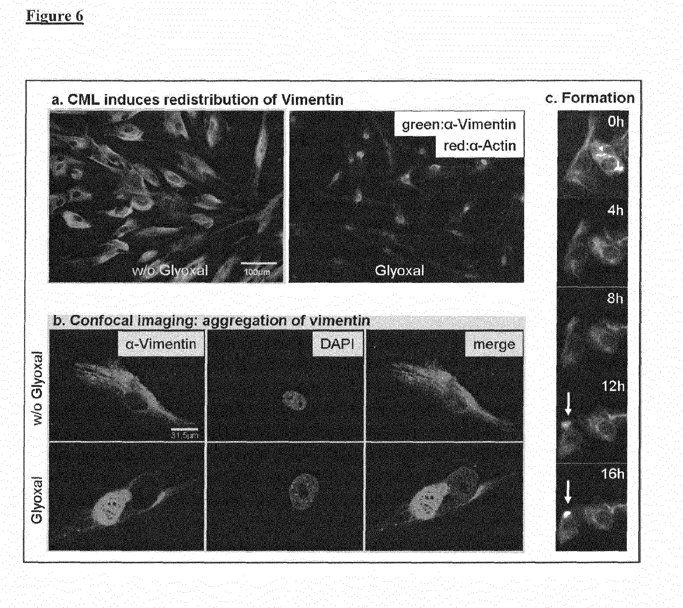

To determine whether the loss of contractile capacity is the result of a CML-induced redistribution of vimentin, immunofluorescence microscopic analysis of fibroblasts with and without glyoxal treatment were employed. Figure 6a demonstrates a strong redistribution of vimentin due to glyoxal treatment, resulting in a perinuclear aggregate of vimentin. About 70% of the human fibroblasts showed a comparable aggregate formation following incubation with 200 µM glyoxal^{18,20,28} for 2 days. About 90% of the fibroblasts were affected after incubation for 7 days.

Fig. 6c displays the time-elapsed rearrangement of the intermediate filament vimentin during glyoxal treatment using fibroblasts expressing vimentin-GFP³³. Rearrangement takes place during the first 16 h of glyoxal treatment. This destruction of the vimentin filament and the localisation of vimentin into an aggregate explains the observed loss of contractile capacity of fibroblasts, since vimentin is significantly involved in the process of contraction²⁵. The distribution of actin seems to be unaffected, reflecting vimentin as the major CML target (Figure 6a). Confocal images of this aggregate (Figure 6b) show a dense vimentin structure inside the aggregate.

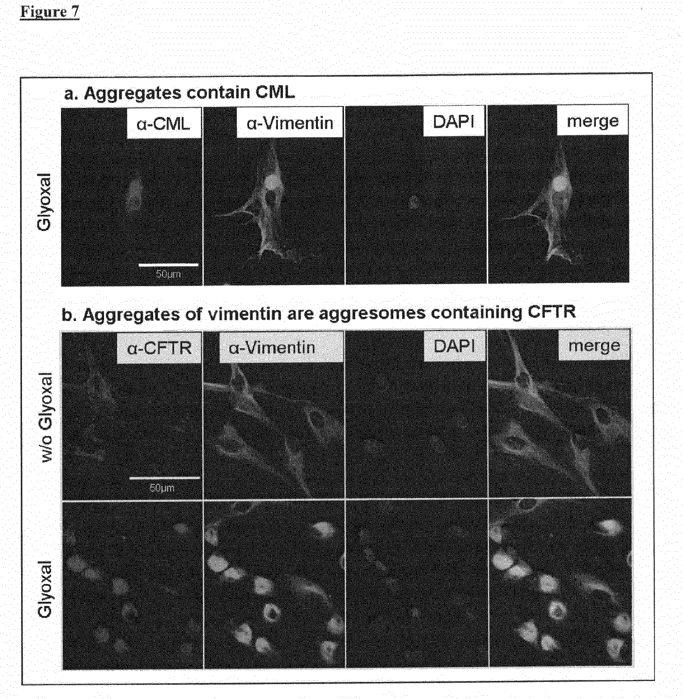

Formation of a vimentin aggregate due to glyoxal treatment raises the question whether the aggregate itself contains CML-modified vimentin. Figure 7a displays the accumulation of CML and vimentin, clearly showing that the majority of the CML signal is located in the perinuclear area and preferentially in the formed vimentin aggregate.

The formation of such a vimentin aggregate near the cell nucleus suggests that CML-vimentin accumulates in aggresomes³². In order to study the formation of typical aggresomal structures vimentin was tested for CFTR^{31,32} distribution in glyoxal treated fibroblasts (Figure 7b) because CFTR is a common marker for aggresomes^{31,32}. Surprisingly, the incubation of human fibroblasts with glyoxal resulted in the redistribution of CFTR into the aggregate. Thus the observed vimentin aggregation contains the aggresome-marker CFTR and can consequently be named aggresome^{31,32,34}. This provides evidence that CML-vimentin represents the damaged protein inside the aggresome, linking the glycation reaction directly to aggresome formation.

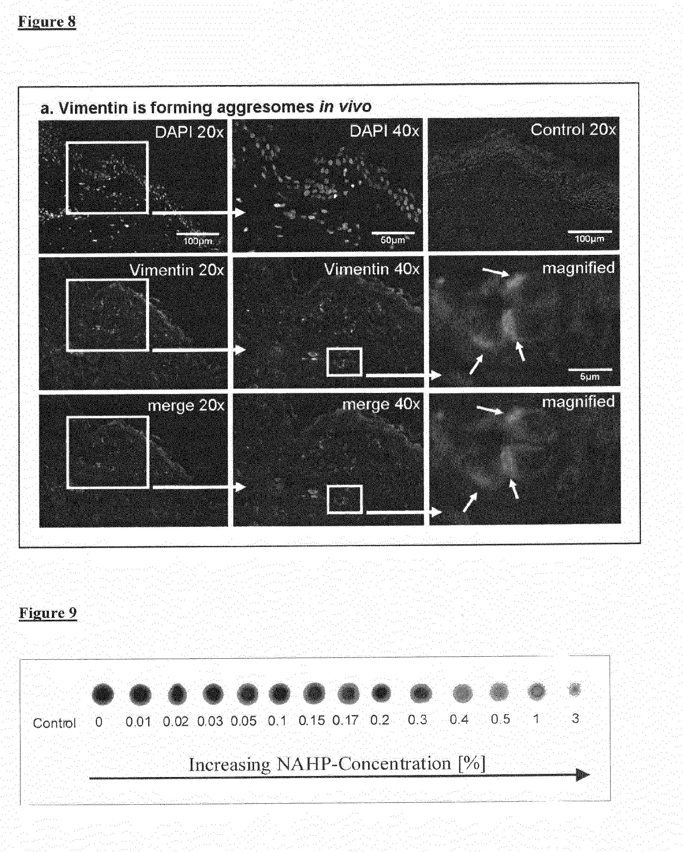

The present inventors also determined the localisation of vimentin and CML in cultured non-glyoxal-treated fibroblasts (Figure 1c). There were no aggresomes detectable in the cells. However CML-staining is located in a more perinuclear area in comparison to total vimentin. Therefore, the question about the *in vivo* relevance of the CML-vimentin aggresomes is important to answer. Accordingly, skin sections obtained from an old human donor (76 years) were investigated. Vimentin was found in the dermal part of the human skin representing a marker for dermal fibroblasts^{35,36} (Figure 8) and in particular, in the form of aggregated vimentin (Figure 8). This confirms *the in vivo* relevance of aggresome formation as a consequence of life-long protein glycation in terms of CML-vimentin.

The experiments described above provide evidence that CML-vimentin is formed in human skin fibroblasts *in vivo.* The structure of vimentin plays a dominant role in the susceptibility of this protein to non-enzymatic glycation, especially when compared to other proteins with longer half-lives and similar expression levels. Until now the longevity of proteins was considered to be among the most important factors in the glycation reaction⁸ due to the non-specific character of the Maillard reaction. Vimentin plays a dominant role in CML-formation, being the major target for this type of glycation in primary human fibroblasts. However, the high degree of CML modification of vimentin is not due to its slow turnover. Although the degradation of actin is slower than that of vimentin, actin, being expressed at a similar rate²², does not show any detectable level of CML adduct formation (Figure 1b). The modification sites of vimentin are predominantly found in its linker regions²⁴, reflecting the exposure of these segments to the surrounding environment. This exposure makes the affected amino acids susceptible to glycation, eventually leading to the structural breakdown of the vimentin filament. In this context Thornalley et al.²³ reported that glycation of albumin by methylglyoxal is enhanced at amino acid residues with high surface exposure causing their accessibility.

Intermediate filaments in general are characterised by a strong structural similarity. This similarity is based on the presence of a centrally located rod domain consisting of the coiled-coil domains 1A, 1 B, 2A, and 2B (Figure 3b). These domains are separated by the linkers L1, L12 and L2²⁶. The linker L1 acts as a flexible hinge to separate the domains 1A and 1B²⁷. This flexibility is required to enable proper dimerisation of intermediate filaments needed for spreading of the filament system. The glycation of the linker region which results in the formation of CML adducts might therefore result in the loss of this flexibility. Herrmann et al.³⁷ demonstrated that the exchange of a single amino acid in the linker regions L1 or L12 is able to block the spreading of various filament systems. Smith et al.²⁶ attributed this fact to the loss of flexibility in the linker regions. The glycation of an amino acid residue in the linker region is additionally affected by the composition of the nearby amino acid residues due to the fact that the reactivity of the residues depends on the availability of free electron pairs on the nitrogen atoms of the amino groups and secondary factors such as steric constraints^{23,38}.

The structural similarity of the family of intermediate filaments suggests that other filament systems like desmin in muscle cells, keratins in epithelial cells or neurofilament triplet proteins in neurons³⁹ could be affected by a high degree of glycation as well. In this regard it is noted that, aggregates of neurofilament proteins-type IV intermediate filaments - were found in Parkinson's disease^{4,40}. Furthermore aggregated type III intermediate filament protein GFAP was reported to aggregate in Alexander's disease⁴¹. It is speculated that the fact that no CML glycation of keratins was detected in primary human keratinocytes (Figure 1a) is due to the fast turnover of keratinocytes, resulting in a shorter life-time of the cells. Additionally, other AGE modifications may be present instead of CML while a strong colocalisation of CML with other AGEs was reported⁴². This colocalisation suggests that glycation of vimentin with AGEs different from CML may be present in the linker regions as well.

CML modification in general contributes to the enhanced stiffness found *in vivo* for skin and other fibroblast containing tissues in ageing⁹. The results set forth above demonstrate that fibroblasts lose their contractile capacity due to glycation of vimentin (Figure 5). A loss of contractile capacity results in the diminished ability of dermal fibroblasts to reorganise collagen fibers. The reorganisation of collagen is essential for tissue development and tissue repair to rearrange the extracellular matrix⁴³. The aggregates of vimentin detected in old human skin constitute strong evidence that vimentin aggregation occurs *in vivo* with all consequences described above. Recent literature reports that vimentin deficient cells exhibit a different mechanical stiffness compared to vimentin containing cells^{44,45}. Inhibition of glycation of vimentin might, therefore, retard the intrinsic ageing of various tissues. Notably vimentin plays a role in wound healing^{25,46} while fibroblasts deficient in vimentin exhibit a reduced ability to reorganise collagen fibres after injury. The impaired wound healing in diabetes may, therefore, partly be based on the high glycation of vimentin according to a increased glycation rate in diabetes in general⁴⁷ due to the higher glucose level. Interestingly CML levels in collagen, which is an extracellular AGE target, were described to be accelerated in diabetes and ageing, leading to further tissue stiffening¹². With regard to wound healing recent findings have demonstrated that the vimentin intermediate filament is significantly involved in the process of lymphocyte adhesion to endothelial cells during diapedesis⁴⁸.

The identification of vimentin as major damaged protein in aggresomes supports the theory that protein aggregates directly enhance ageing of tissues³⁴, linking ageing to glycation. The aggregation of CML-modified proteins inside the aggresome and thus the separation of highly glycated proteins from the remaining protein pool may represent a cellular attempt to minimise the damage for nonglycated, functional active proteins. This proposal is supported by the fact that AGEs different from CML, like pentosidines or crosslines, cross-link proteins⁸, which obviously results in damage to surrounding proteins.

An extraordinary production of vimentin was reported previously for senescent fibroblasts⁴⁹. This may be a cellular response to compensate the damaged CML-vimentin resulting in the *de novo* production of vimentin to maintain function. The present inventors were able to show that exemplarily *in vivo,* vimentin aggresomes were found along with distributed vimentin throughout the cytosol. This indicates a chronic process of vimentin modification during the life-time of the donor, and in parallel the adaptation of the fibroblast vimentin expression to this condition.

The present inventors furthermore have found that the formation of CML vimentin can be inhibited under physiological conditions by N-acetylhydroxyproline and structurally related compounds (which are disclosed in co-pending application entitled "COSMETIC AND PHARMACEUTICAL APPLICATIONS OF N-ACYLATED AMINO ACIDS AND STRUCTURALLY RELATED COMPOUNDS", filed concurrently herewith, the entire disclosure whereof is incorporated by reference herein) and have also found that N-acetylhydroxyproline and structurally related compounds, when incorporated into cosmetic or pharmaceutical (dermatological) compositions, provide even further unexpected and beneficial advantages.

### SUMMARY OF THE INVENTION

The present invention provides a method (for example, a cosmetic method) of preventing or (significantly) reducing the formation of aggresomes in fibroblasts of a (preferably human) subject. The method comprises the administration to the subject of N-acetylhydroxyproline in an amount which is effective to (substantially) prevent or (significantly) reduce non-enzymatic glycation of vimentin in the fibroblasts, thereby (substantially) preventing or (significantly) reducing the formation of aggresomes in the fibroblasts.

In one aspect of this method, the amount of N-acetylhydroxyproline may be effective to (substantially) prevent or (significantly) reduce the formation of CML adducts in one or more linker regions of vimentin.

In another aspect of the method, the N-acetylhydroxyproline may be administered by topical application of a dermatological or cosmetic composition which comprises N-acetylhydroxyproline.

In yet another aspect of the method, the composition may provide or maintain a pH of from about 7.0 to about 7.4 (e.g., from about 7.1 to about 7.3 such as, e.g., about 7.2).

The present invention also provides a method (e.g., a cosmetic method) of preventing or (significantly) reducing the loss of contractile capacity of fibroblasts in a (preferably human) subject. The method comprises an administration to the subject of N-acetylhydroxyproline in an amount which is effective to (substantially) prevent or (significantly) reduce the non-enzymatic glycosylation of vimentin in the fibroblasts. Preferably, the administration comprises a topical administration.

The present invention also provides a method (e.g., a cosmetic method) of preventing or (significantly) reducing the intrinsic ageing of tissues in a (preferably human) subject. The method comprises an administration to the subject of N-acetylhydroxyproline in an amount which is effective to (substantially) prevent or (significantly) reduce the non-enzymatic glycosylation of vimentin in fibroblasts of the subject.

The present invention also provides a method of improving wound healing in a (preferably human) subject in need thereof. The method comprises an administration to the subject of N-acetylhydroxyproline in an amount which is effective to (significantly) reduce the non-enzymatic glycosylation of vimentin in fibroblasts of the subject.

In one aspect of the method, the subject may exhibit an increased level of glucose. For example, the subject may suffer from diabetes.

The present invention also provides a method of protecting structural proteins of a (preferably human) subject from glycation. The method comprises administering to the subject an effective amount of N-acetylhydroxyproline.

The present invention also provides a method of (substantially) preventing or (significantly) reducing the formation of AGEs in a living organism (for example, a human body). The method comprises providing the organism with an effective amount of N-acetylhydroxyproline.

In one aspect of the method, the AGEs may comprise one or more of a carboxymethyl lysine (CML) adduct, a carboxyethyl lysine (CEL) adduct and a fructose-lysine adduct.

The present invention also provides a method of (substantially) preventing or (significantly) reducing the glycation of lysine residues of a protein in a living organism (for example, in the human body). The method comprises providing the organism with an effective amount of N-acetylhydroxyproline.

In one aspect of the method, the protein may comprise at least one of tau, neurofilament protein and αB crystallin.

In another aspect, the protein may comprise an extracellular matrix protein. For example, the extracellular matrix protein may comprise collagen and/or elastin.

In yet another aspect of the method, the protein may comprise a filamentous protein such as, e.g., keratin and/or desmin.

In a still further aspect of the method, the protein may comprise an immunoglobulin or an enzyme.

In another aspect, the protein may comprise albumin.

In another aspect, the protein may comprise vimentin and/or plectin.

The present invention also provides a method of (substantially) preventing or (significantly) reducing the formation of aggresomes in cells of a living organism (for example, the human body). The method comprises providing the cells with an effective amount of N-acetylhydroxyproline.

In one aspect of the method, the aggresomes may comprise a CML adduct of vimentin.

The present invention also provides a method of substantially preventing or delaying the onset of, or substantially preventing or retarding the progression of a condition which is associated with the formation of AGEs in a (preferably human) subject in need thereof. The method comprises administering to the subject an effective amount of N-acetylhydroxyproline.

In one aspect of the method, the condition may comprise a neurodegenerative disease such as, e.g., Parkinson's disease, Alzheimer's disease, amyotrophic lateral sclerosis (ALS), vascular dementia, Alexander's disease or diffuse Lewy body disease.

In another aspect of the method, the condition may comprise an age-related disease such as, e.g., atherosclerosis, arterogenesis, arterial stiffening, pulmonary fibrosis or male erectile dysfunction.

In yet another aspect, the condition may comprise inflammation, glomerular sclerosis, cardiac hypertrophy and/or endothelial dysfunction.

The present invention also provides a method of retarding normal ageing in a (preferably human) subject. The method comprises administering to the subject an effective amount of N-acetylhydroxyproline.

The present invention also provides a method of substantially preventing or (significantly) ameliorating a complication caused by diabetes in a (preferably human) subject in need thereof. The method comprises administering to the subject an effective amount of N-acetylhydroxyproline. For example, N-acetylhydroxyproline may be administered orally or parenterally and/or it may be administered in combination with a diabetes drug such as, e.g., insulin.

In one aspect of the method, the complication may comprise retinopathy, cataract formation, nephropathy, impaired wound healing and/or an increased risk of acute myocardial infarction.

The present invention also provides a method of retarding the (intrinsic and/or extrinsic) ageing of skin and skin appendages such as nails and hair (e.g., retarding or substantially preventing the formation of wrinkles and/or age spots, the loss of skin elasticity, the development of dry and/or chapped skin, cellulite, etc.) and/or a method of improving skin quality in general in a (preferably human) subject. The method comprises administering to the subject an effective amount of N-acetylhydroxyproline.

In one aspect of the method, one or more of the loss of skin elasticity, the formation of wrinkles, the formation of age spots and the development of dry skin may be retarded.

In another aspect of the method, the administration may be carried out topically and at about cellular pH, i.e., at a pH of from about 7.0 to about 7.4, e.g., from about 7.1 to about 7.3, such as about 7.2. This aspect is of interest particularly for achieving an intracellular effect ("AGE-Detox"), in particular for long-term application, protein reactivation in the skin ("Youth your AGE"), etc.

In yet another aspect of the method, the administration may be carried out topically and at a pH of from about 4.8 to about 5.2, e.g., from about 4.9 to about 5.1, such as about 5.0. This aspect is of interest in particular for achieving an extracellular effect ("pH-Detox"), for example, in connection with, e.g., "peeling" and beautification/beautifying purification of the skin.

In another aspect of the methods of the present invention, N-acetylhydroxyproline may be administered orally. In yet another aspect, it may be administered parenterally.

In a still further aspect, NAHP may be applied topically to the skin.

The present invention also provides a cosmetic or pharmaceutical (e.g. dermatological) composition which comprises N-acetylhydroxyproline and at least one other compound which is capable of inhibiting the formation of AGEs and/or breaking cross-links of peptides affected by AGE formation. For example, the at least one other compound may be selected from one or more of aminoguanidine, carnosine, metformin, acarbose, pyridoxamine, 2,3-diaminophenazone, tenilsetam (3-2-thienyl-2-piperazinone), scriptaid (6-(1,3-dioxo-1H,3H-benzo[de]isoquinolin-2-yl)-hexanoic acid hydroxyamide), DPDTB (1,5-diphenyl-2,4-dithiobiuret), OPB-9195 (2-isopropylidenehydrazono-4-oxo-thiazolidin-5yl-acetanilide), pentoxifylline, pioglitazone, kinetin (furfuryladenine) and ALT 711 (4,5-dimethyl-3[2-oxo-2-phenylethyl-thiazoliumchloride).

The present invention also provides a method of increasing the effectiveness of an acidic composition which is capable of preventing or reducing formation of AGEs in (preferably human) skin, wherein the method comprises including in the composition N-acetylhydroxyproline.

In one aspect of the method, the composition may have a pH of from about 4.8 to about 5.2, for example a pH from about 4.9 to about 5.1 such as, e.g., a pH of about 5.0.

The present invention also provides a cosmetic or pharmaceutical (e.g., dermatological) composition which comprises N-acetylhydroxyproline in combination with one or more substances selected from one or more of the following groups (a) to (e):
(a) starch-based particles which have an average particle size of from about 1 µm to about 100 µm;
(b) emulsifiers comprising at least one carbohydrate moiety;
(c) thickeners selected from one or more of acryloyldimethyltaurate/vinylpyrrolidone copolymers, acrylic acid/vinylpyrrolidone crosspolymers, polyacrylates and polyesters;
(d) UV filters selected from one or more of 2,4-bis(((2-ethylhexyloxy)-2-hydroxyl)-phenyl)-6-(4-methoxyphenyl)-(1,3,5)-triazine, terephthalidene dicamphor sulfonic acid, 2,2'-methylene-bis-6-(2H-benzotriazol-2-yl)-4-(tetramethylbutyl)-1,1,3,3-phenol, diethylamino hydroxybenzoyl hexyl benzoate and 2,2'-(1,4-phenylene)-bis(1H-benzimidazole-4,6-disulfonic acid, monosodium salt);
(e) skin benefit agents selected from one or more of flavonoids, in particular rutin and derivatives thereof (for example α-glycosylrutin such as α-glucosylrutin), Coenzyme Q10, creatine, L-carnitine, genistein, daidzein, licochalcone A, folic acid and vitamin B3 (niacinamide).

In one aspect, the composition may further comprise at least one other compound which is capable of at least one of inhibiting the formation of AGEs and breaking cross-links of peptides affected by AGE formation.

The present invention also provides a method of improving the application properties and/or the sensory properties of a cosmetic or dermatological composition. The method comprises including in the cosmetic or dermatological composition N-acetylhydroxyproline in an amount which is effective for improving at least one application property (e.g., the spreadability and/or absorbability) and/or at least one sensory property (e.g., reduced sticky feeling) of the composition.

The present invention also provides a method of boosting the UV protection properties of a composition for topical application to skin which comprises at least one (preferably organic) UV filter substance. The method comprises including in the composition N-acetylhydroxyproline in an amount which is effective for boosting the UV protection properties of the composition. For example, the method of the present invention may increase the SPF (sun protection factor) and/or the UVA-SPF and/or the ratio of UVB protection vs. UVA protection. A suitable amount of N-acetylhydroxyproline may be in the range of, for example, from about 0.1 % by weight to about 30 % by weight and a suitable increase may be by at least about 2 %, e.g., by at least about 5 %, or by at least about 8 %.

The present invention also provides a method of reducing or substantially eliminating undesired or uneven pigmentation of the skin. The method comprises applying to the skin affected by the undesired or uneven pigmentation N-acetylhydroxyproline.

The present invention also provides a method of reducing or substantially eliminating deficient skin associated with menopause, post-menopause or andropause (e.g., PADAM) in a human (such as, e.g., dark circles under the eyes, sagging skin, sagging cheeks, etc.). The method comprises applying to the deficient skin N-acetylhydroxyproline.

The present invention also provides a method of deregulating the fat metabolism in skin. The method comprises applying to the skin N-acetylhydroxyproline.

The present invention also provides a method of reducing or substantially preventing the formation of AGEs in epidermal or dermal stem cells of a (preferably human) subject. The method comprises administering to the subject N-acetylhydroxyproline.

The present invention also provides a method of reducing or substantially preventing itching skin in a subject. The method comprises applying to the itching skin of the subject N-acetylhydroxyproline.

The present invention also provides a method of protecting a cosmetic or pharmaceutical (e.g., dermatological) composition from (atmospheric) oxidation or photooxidation. The method comprises including in the composition N-acetylhydroxyproline.

The present invention also provides a method of stabilizing urea in a cosmetic or pharmaceutical (e.g., dermatological) composition.The method comprises including in the composition N-acetylhydroxyproline.

### DETAILED DESCRIPTION OF THE PRESENT INVENTION

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the several forms of the present invention may be embodied in practice.

The term "significantly reducing" as used herein denotes a reduction which is statistically significant and will usually be a reduction by least about 20 %, e.g., by least about 40 %, by least about 50 %, by least about 70 % or by least about 90 % in comparison to an untreated subject. The conditions under which the comparison is carried may be physiological conditions or non-physiological conditions such as, e.g., an elevated temperature (i.e., a temperature above body temperature). The period over which the comparison is to be carried out depends on the effect to be measured and the conditions employed and may, for example, be about 24 hours, about one week, about one month or even longer.

Further, it is noted that whenever the terms "N-acetylhydroxyproline" or "NAHP" are used in the present specification and the appended claims these terms encompass N-acetyl-3-hydroxyproline and N-acetyl-4-hydroxyproline both individually and in any combinations thereof. N-Acetyl-4-hydroxyproline is the preferred isomer for use in the present invention.

In addition to its advantageous properties with respect to the prevention or reduction of non-enzymatic glycosylation, N-acetylhydroxyproline is also useful for other purposes which may or not be connected with non-enzymatic glycosylation. For example, N-acetylhydroxyproline is useful for improving, *inter alia,* uneven and undesired pigmentation of skin and skin appendages such as, e.g., age spots (senile lentigo), freckles and post-inflammatory hyperpigmentation (in this regard, see Example 37 below). While not wishing to be bound by any theory, it is speculated that these beneficial effects may also be due to the AGE inhibiting effect of N-acetylhydroxyproline. In particular, AGEs are a heterogeneous mixture of protein-bound sugars and sugar fragments which accumulate in the human body over time (i.e., by aging). The formation of AGEs is caused by the Maillard reaction and results in products a part of which exhibits a characteristic brown color. The present inventors also have found that the formation of AGEs results in a significant change in cell functions, which may also play a role in pigmentation disorders.

N-Acetylhydroxyproline is also useful for protecting (epidermal and dermal) stem cells from the formation of AGEs and thus, helps to regenerate tissue and/or retard the progression of tissue damage. Because a stem cell is present in the body for a particularly long period of time before it dies, the probability of the formation of AGEs is increased in this case.

NAHP also is useful for maintaining or restoring the barrier properties of skin and for counteracting moisture loss of skin. NAHP further is useful for protecting skin from protein damage and environmental influences. In particular, N-acetylhydroxyproline helps to maintain the structure of typical water-binding proteins of the dermis and epidermis and of barrier-supporting proteins and lipids of the stratum corneum. NAHP is effective for maintaining or even increasing the moisture content of the skin, even for extended periods of time (several days or even longer).

Further, N-acetylhydroxyproline is useful for deregulating the fat metabolism of skin. For example, NAHP is effective for retarding cellulite and/or for improving symptoms of cellulite and also is effective for improving or substantially eliminating stretchmarks.

Even further, N-acetylhydroxyproline is effective for treating skin imperfections such as cracks, scars and striae (e.g., caused by pregnancy), which result in a noticeable change in the appearance of the cutis and subcutis. Additionally, NAHP may also be used for treating or preventing irritative/inflammatory skin phenomena such as sunburn or razor burn.

NAHP may be administered/applied in any conventional way such as by, e.g., oral (including sublingual, buccal), parenteral and topical administration. The corresponding compositions will usually comprise a (non-toxic) cosmetically and/or pharmaceutically acceptable carrier or diluent. Non-limiting examples of corresponding pharmaceutical dosage forms include powders, tablets, capsules, suppositories, injections, drops, syrups, sublingual tablets, troches, and lozenges. Ointments and gels can advantageously be utilized for topical administration. Especially in the case of a condition such as diabetes NAHP may be administered (e.g., orally or parenterally) in combination with a diabetes drug such as, e.g., insulin.

Non-limiting examples of forms of compositions for topical cosmetic applications include various types of emulsions (O/W, W/O, O/W/O, W/O/W, W/S, S/W, etc.), hydrodispersions, creams, gels, sticks and aerosols (sprays).

Liquid preparations such as syrups that are suitable for oral administration can be produced using water, sugars such as lactose, glucose, mannitol, sucrose, sorbitol and fructose, glycols such as polyethylene glycol and propylene glycol, vegetable oils such as peanut oil, olive oil, soybean oil sesame oil, antiseptics such as parabens, etc.

Furthermore, tablets, powders and granules can be produced using conventionally used excipients such as lactose, corn starch, sodium alginate, agar, pectin, acacia, magnesium stearate, talc, polyvinyl alcohol, hydroxypropyl cellulose, gelatin, fatty acid esters, glycerin, to name but a few. Additionally, the carrier or diluent may include a material for controlled (e.g., delayed and/or extended) releasesuch as glyceryl monostearate or glyceryl distearate with or without a wax.

Parenteral dosage forms which are useful for subcutaneous, intramuscular or intravenous administration may also be employed advantageously. To enhance their stability the composition can be frozen after filling into a vial and the water removed under vacuum. The dry lyophilized powder is then sealed in the vial and an accompanying vial of water for injection is supplied to reconstitute the powder immediately prior to use. Preparations appropriate for parenteral administration comprise, preferably, a sterilized aqueous agent containing an active compound, which is isotonic to the recipient's blood. In the case of an injection, for example, an injectable solution is prepared using a carrier consisting of a salt solution, a glucose solution or a mixture of saline and a glucose solution.

NAHP and NAHP containing compositions may further be applied by mechanical devices. For example, they may be applied by sonophoresis systems, iontophoresis systems, and by means of mechanical massage devices (e.g., for throbbing, vacuum, roll, pressure or brush massage) or massage combs.

NAHP is employed in an amount which is sufficient to treat or alleviate the particular condition, disorder etc., that a subject is afflicted with. For administration to a subject, NAHP may advantageously be administered daily in amounts ranging from about 5 to about 100 milligrams per kilogram. In humans, NAHP is preferably administered in an amount of from about 2 milligrams to about 20 milligrams per kilogram of body weight per day. In the case of parenteral administration such as intravenous administration, NAHP may, for example, be administered in a dose of from about 0.5 to about 5000 mg, e.g., from about 5 to about 1000 mg, or from about 50 to about 500 mg per adult once to several times a day. It should be understood, however, that although preferred dosage ranges are given, the dose level for a given patient depends upon several factors such as the condition or disorder to be treated, etc. Additionally, many other factors which modify the actions of drugs must be taken into account by those skilled in the therapeutic use of medicinal agents, as for example, age, body weight, sex, diet, time of administration, route of administration, rate of excretion, drug combination, reaction sensitivities and severity of the disease.

A pharmaceutical composition will usually comprise NAHP in a concentration of from about 2 to about 1000 mg/g, e.g., from about 5 to about 500 mg/g, or from about 50 to about 200 mg/g of pharmaceutical composition.

The content of N-acetylhydroxyproline in a cosmetic or pharmaceutical (e.g., dermatological) composition may vary over a wide range depending on the desired effect. By way of non-limiting example, the concentration of N-acetylhydroxyproline in a cosmetic or dermatological composition may be from about 0.001 % to about 30 % by weight, e.g., from about 0.01 % to about 15 % by weight, of from about 0.1 % to about 2 % by weight. Often, the amount of NAHP in a cosmetic or pharmaceutical (e.g.,dermatological) composition will be not higher than about 20 %, e.g., not higher than about 10 %, or not higher than about 5 % by weight, and not lower than about 0.05 %, e.g., not lower than about 0.1 %, or not lower than about 0.5 % by weight, relative to the total weight of the composition.

In addition to N-acetylhydroxyproline (optionally in combination with one or more structurally related compounds which are disclosed in the copending application referred to above), a cosmetic or dermatological composition may, for example, comprise conventional cosmetic ingredients. Examples of conventional ingredients include solids (e.g., inorganic and/or organic powders), fats, oils, waxes, moisturizers, emollients, water soluble polymers, oil soluble polymers, surfactants, inorganic and organic pigments, coloring agents such as organic dyestuffs, organic solvents such as ethanol, glycerol, ultraviolet ray absorbents, antiseptics, antioxidants, pigments, thickeners, pH regulators, emulsifiers, perfumes, antiperspirants, fungicides, antimicrobial agents, skin softeners, preservatives, water, to name but a few.

Non-limting examples of moisturizers which are suitable for use in cosmetic or dermatological compositions according to the present invention include glycerin, lactic acid and salts thereof, in particular, sodium lactate, butylene glycol, propylene glycol, biosaccharide gum-1, glycine soja, ethylhexyloxyglycerin, pyrrolidone carboxylic acid, and urea. Further examples of suitable moisturizers include polymeric moisturizers from the group of water-swellable or water-gelable polysaccharides such as hyaluronic acid, chitosan and a fucose-rich polysaccharide which is filed in Chemical Abstracts under the registry No. 178463-23-5 and is commercially available from SOLABIA S.A. under the trade name Fucogel®.

Moiturizers may also advantageously be used as anti-wrinkle agents for treating and preventing cosmetic or dermatological changes of the skin as they occur, for example, during skin ageing. A particularly suitable moisturizer is glycerin, preferably in concentrations of from about 0.05 % to about 30 % by weight, in particular from about 1 % to about 10 % by weight, relative to the total weight of the composition.

The present inventors have identified several auxiliaries and additives, especially for cosmetic or dermatological compositions, which are particularly suitable for increasing one or more of the bioavailability, effectiveness and stability of N-acetylhydroxyproline. These additives and auxiliaries include, for example:
(a) starch-based particles which have an average particle size of from about 1 µm to about 100 µm (e.g., from about 10 µm to about 40 µm) and are preferably substantially non-swellable in water such as, e.g., particles which comprise one or more of tapioca starch, aluminum starch octenylsuccinate (commercially available, for example under the trade name Staare 116), sodium starch octenylsuccinate and distarch phosphate (commercially available, for example, under the trade name Siam OP);
(b) emulsifiers comprising at least one carbohydrate (e.g., glucose) moiety such as, e.g., polyglyceryl-3-methylglucose distearate, commercially available, for example, under the tradename Tegocare® 450, and cetearyl glucoside, commercially available, for example, under the trade name Emulgator CG-90;
(c) thickeners selected from one or more of (e.g., ammonium) acryloyldimethyltaurate/vinylpyrrolidone copolymers, commercially available, for example, under the trade name Aristoflex® AVC, acrylic acid/vinylpyrrolidone crosspolymers, commercially available, for example, under the trade name Ultrathix®, polyacrylates (e.g., polyacrylate-3) and polyesters (e.g., polyester-5);
(d) inorganic and organic UV filters, preferably organic UV filters and in particular, UV filters selected from one or more of bemotrizinol (commercially available, e.g., under the trade name Tinosorb® S from Ciba), 2,2'-methylene-bis-6-(2H-benzotriazol-2-yl)-4-(tetramethylbutyl)-1,1,3,3-phenol (commercially available, e.g., under the trade name Tinosorb® M from Ciba), diethylamino hydroxybenzoyl hexyl benzoate (commercially available, e.g., under the trade name Uvinul® A Plus from BASF) and 2,2'-(1,4-phenylene)-bis(1H-benzimidazole-4,6-disulfonic acid, monosodium salt), commercially available, for example, under the trade name NeoHeliopan® AP from Symrise;
(e) skin benefit agents selected from one or more of bioquinones, in particular, ubiquinone (coenzyme Q10) and ubiquinol, folic acid and derivatives thereof, in particular, tetrahydrofolic acid and dihydrofolic acid, niacin and derivatives thereof, in particular, niacinamide (vitamin B₃), creatine and creatinine, carnitine, in particular, L-carnitine, flavonoids, in particular rutin and derivatives thereof (for example α-glycosylrutin such as α-glucosylrutin), isoflavone, genistein, daidzein, biotin, cardiolipin, lipoic acid, lipamide, anti-freezing proteins, pimpinella-anis fruit extract, hop extract and hop-malt extract, primrose, calcium, pomegranate, arctiin, glycerrhetic acid, oxygen-containing and oxygen-enriched formulations, agents which promote a restructuring of the connective tissue such as natural and synthetic isoflavonoids and isoflavonoid-containing plant extracts (e.g., soy and clover extracts), agents which support skin functions in dry skin such as ascorbic acid, propionic acid, glycerylglucose, green tea extract, eucalyptus oil, urea and mineral salts such as NaCl, sea minerals and osmolytes such as, e.g., inositol, betain and quaternary ammonium compounds, agents for alleviating or positively influencing irritative conditions in the case of sensitive skin in general and in the case of skin irritated by noxae such as UV radiation or chemicals, such as sericosides, various extracts of liquorice, in particular, licochalcone A, silymarin, silyphos, dexpanthenol, inhibitors of the prostaglandin metabolism, in particular, inhibitors of cyclooxygenase, inhibitors of the leucotriene metabolism, in particular, inhibitors of 5-lipoxygenase and 5-lipoxygenase-inhibitor protein, FLAP, pigmentation modulators which reduce skin pigmentation such as tyrosine sulfate, dioic acid (8-hexadecene-1,16-dioic acid), kojic acid, hydroquinone, arbutin, alpha-arbutin, deoxyarbutin, fruit acids, in particular, alpha-hydroxyacids (AHAs), bearberry extract, ursolic acid, aminoguanidine, pyridoxamine, inhibitors of proteinase activated receptor 2 (PAR-2), agents which cause an accelerated and/or enhanced tanning of the skin with or without the influence of UV radiation such as nucleic acid oligonucleotides, purines and pyrimidines, NO releasing substances, tyrosine and derivatives thereof, in particular, N-acetyl-tyrosine, phenylalanine and derivatives thereof, in particular, N-acetyl-phenylalanine, anti-wrinkle agents such as flavone glycosides, in particular, alpha-glycosylrutin, vitamin E and derivatives thereof, and argania spinosia leaf extract.

According to the present invention, the above and other agents may be present, for example, in encapsulated form. Non-limiting examples of encapsulation materials include collagen matrices, cyclic oligosaccharides, in particular, alpha-, beta-, HP-beta, random-Me-beta, gamma-cyclodextrins, cellulose, gelatine, wax and liposomes.

Cosmetic or dermatological compositions may take various forms such as, e.g., powders, creams, lotions, foams, milks, gels, emulsions and shampoos.

Non-limiting examples of cosmetic compositions include hair dying compositions (e.g., as a shampoo formulation), hair rinse compositions, permanent wave compositions, anti-hair loss compositions, compositions for strengthening the hair and/or hair roots, conditioners, styling compositions, and anti-dandruff compositions, decorative cosmetics such as, e.g., nail varnishes, lipsticks, and foundations, washing and shower compositions, and creams and emulsions for skin care.

Examples 1 to 33 below illustrate exemplary embodiments of NAHP-containing cosmetic or dermatological compositions in accordance with the present invention.

### Example 1: O/W Emulsions

| | **1** | **2** |
|---|---|---|
| **Ingredient (INCI)** | **% by weight** | |
| Polyglyceryl-3-methyl glucose distearate | 2.00 | 2.00 |
| Stearyl alcohol | 2.00 | 2.00 |
| C₁₂₋₁₅ Alkyl benzoate | 3.00 | 3.00 |
| Butylene glycol dicaprylate/dicaprate | 2.00 | 2.00 |
| Cyclomethicone | 3.00 | 3.00 |
| Hydrogenated polydecene | 2.00 | 2.00 |
| Dimethylpolysiloxane (dimethicone) | 1.00 | 1.00 |
| Petrolatum | 1.00 | 1.00 |
| TiO₂ | 1.00 | 1.00 |
| N-Acetylhydroxyproline | 1.00 | 2.00 |
| Allantoin | 0.10 | 0.10 |
| Phenoxyethanol | 0.40 | 0.40 |
| Iodopropynylbutyl carbamate | 0.05 | 0.05 |
| p-Hydroxybenzoic alkyl ester (paraben) | 0.20 | 0.20 |
| Xanthan gum | 0.10 | 0.10 |
| Carbomer | 0.10 | 0.10 |
| Butylene glycol | 3.00 | 3.00 |
| Additives (talc, BHT) | 0.50 | 0.50 |
| Perfume | q.s. | q.s. |
| Water | ad 100 | ad 100 |

### Example 2: O/W Emulsions

| | **1** | **2** |
|---|---|---|
| **Ingredient (INCI)** | **% by weight** | |
| Polyglyceryl-3-methyl glucose distearate | 3.00 | 3.00 |
| Cetyl alcohol | 1.00 | 1.00 |
| Cyclomethicone | 3.00 | 3.00 |
| Dicapryl ether | 2.00 | 2.00 |
| Paraffinum liquidum | 3.00 | 3.00 |
| Ethylenediaminetetraacetic acid trisodium | 0.10 | 0.10 |
| Carbomer | 0.10 | 0.10 |
| Hydroxypropylmethylcellulose | 0.30 | 0.30 |
| N-Acetylhydroxyproline | 0.50 | 1.50 |
| Aluminum chlorohydrate | 5.00 | 5.00 |
| Glycerin | 3.00 | 3.00 |
| NaOH | q.s. | q.s. |
| Preservatives | q.s. | q.s. |
| Perfume | q.s. | q.s. |
| Water | ad 100 | ad 100 |

### Example 3: O/W Emulsions

| | **1** | **2** |
|---|---|---|
| **Ingredient (INCI)** | **% by weight** | |
| Polyglyceryl-3-methyl glucose distearate | 3.00 | 3.00 |
| Sorbitan stearate | 1.00 | 1.00 |
| C₁₂₋₁₅ Alkyl benzoate | 2.00 | 2.00 |
| Ethylhexyl coconut fatty acid ester | 5.00 | 5.00 |
| Octamethyltetrasiloxane (cyclomethicone) | 5.00 | 5.00 |
| Polydecene | 1.00 | 1.00 |
| N-Acetylhydroxyproline | 1.00 | 1.50 |
| Tocopherol | 0.10 | 0.10 |
| EDTA | 0.20 | 0.20 |
| para-Hydroxybenzoic alkyl ester (paraben) | 0.40 | 0.40 |
| Methylpropanediol | 3.00 | 3.00 |
| Ethanol, denaturated | 2.00 | 2.00 |
| Xanthan gum | 0.20 | 0.20 |
| Carbomer | 0.10 | 0.10 |
| Glycerin | 5.00 | 5.00 |
| Filler/additives (distarch phosphate, talc) | 2.00 | 2.00 |
| Perfume | q.s. | q.s. |
| Water | ad 100 | ad 100 |

### Example 4: O/W Emulsions

| | **1** | **2** |
|---|---|---|
| **Ingredient (INCI)** | **% by weight** | |
| Glyceryl stearate citrate | 1.50 | 1.50 |
| Cetyl stearyl alcohol | 1.00 | 1.00 |
| Caprylic/capric triglyceride | 1.00 | 1.00 |
| Dicaprylcarbonate | 2.00 | 2.00 |
| Dimethylpolysiloxane, cyclic (dimethicone) | 4.00 | 4.00 |
| Carbopol | 0.15 | 0.15 |
| Acrylic acid/C 10-30 alkylmethacrylate-copolymer | 0.25 | 0.25 |
| Dimethicone | 0.75 | 0.75 |
| Jojoba oil | 1.00 | 1.00 |
| Tocopheryl acetate | 0.75 | 0.75 |
| Glycerin | 10.00 | 10.00 |
| Ethanol | 1.00 | 1.00 |
| N-Acetylhydroxyproline | 1.00 | 2.50 |
| Fillers/additives (distarch phosphate, BHT, talc, aluminum starch octenylsuccinate, cyclodextrin) | 1.00 | 1.00 |
| Perfume | q.s. | q.s. |
| Preservatives | q.s. | q.s. |
| Water | ad 100 | ad 100 |

| | | |
|---|---|---|
| pH value adjusted to 6.0 | | |

### Example 5: O/W Emulsions

| | **1** | **2** |
|---|---|---|
| **Ingredient (INCI)** | **% by weight** | |
| Glyceryl stearate citrate | 3.00 | 3.00 |
| Stearyl alcohol | 1.00 | 1.00 |
| Caprylic/capric triglyceride | 1.00 | 1.00 |
| Octyldodecanol | 1.00 | 1.00 |
| Dicaprylether | 1.00 | 1.00 |
| N-Acetylhydroxyproline | 0.50 | 1.00 |
| Carbomer | 0.15 | 0.15 |
| Glycerin | 3.00 | 3.00 |
| Perfume, preservatives, colorants, antioxidants, etc. | q.s. | q.s. |
| Water | ad 100 | ad 100 |

| | | |
|---|---|---|
| pH value adjusted to 5.5 | | |

### Example 6: O/W Emulsions

| | **1** | **2** |
|---|---|---|
| **Ingredient (INCI)** | **% by weight** | |
| Glyceryl stearate citrate | 3.00 | 3.00 |
| Cetyl stearyl alcohol | 1.00 | 1.00 |
| Cyclomethicone | 4.00 | 4.00 |
| Octyldodecanol | 1.00 | 1.00 |
| Dimethicone | 1.00 | 1.00 |
| N-Acetylhydroxyproline | 1.00 | 1.50 |
| Allantoin | 0.10 | 0.10 |
| Citric acid, sodium salt | 0.10 | 0.10 |
| Ethanol, denaturated | 3.00 | 3.00 |
| Aluminum zirconium chlorohydrate | 0.50 | 0.50 |
| Ammoniumacryloyl dimethyltaurate/VP copolymer | 0.30 | 0.30 |
| Glycerin | 10.00 | 10.00 |
| Additives (distarch phosphate, SiO₂, talc) | 0.1 | 0.1 |
| Perfume, preservatives, antioxidants, etc. | q.s. | q.s. |
| Water | ad 100 | ad 100 |

| | | |
|---|---|---|
| pH value adjusted to 5.5 | | |

### Example 7: O/W Emulsions

| | **1** | **2** | **3** |
|---|---|---|---|
| **Ingredient (INCI)** | **% by weight** | | |
| Glyceryl stearate citrate | 2.00 | 2.00 | 2.00 |
| Cetyl alcohol | 1.00 | 1.00 | 1.00 |
| Cyclomethicone | 3.00 | 3.00 | 3.00 |
| Jojoba oil | 0.30 | 0.30 | 0.30 |
| Paraffinium liquidum | 1.00 | 1.00 | 1.00 |
| N-Acetylhydroxyproline | 0.25 | 0.50 | 0.75 |
| Glycerin | 3.00 | 3.00 | 3.00 |
| Serine | 0.10 | 0.10 | 0.10 |
| Tocopherol acetate | 1.00 | 1.00 | 1.00 |
| Xanthan gum | 0.10 | 0.10 | 0.10 |
| Perfume, preservatives, colorants, antioxidants, etc. | q.s. | q.s. | q.s. |
| Water | ad 100 | ad 100 | ad 100 |

| | | | |
|---|---|---|---|
| pH value adjusted to 6.0 | | | |

### Example 8: O/W Emulsion

| **Ingredient (INCI)** | **% by weight** |
|---|---|
| PEG-40 Stearate | 1.00 |
| Glyceryl Stearate | 2.50 |
| C₁₂₋₁₅ Alkyl Benzoate | 1.00 |
| Octyldodecanol | 0.50 |
| Cetearyl Alcohol | 2.50 |
| Simmondsia Chinensis | 0.50 |
| Glycerin | 5.00 |
| Ethylhexyl Methoxycinnamate + BHT | 7.50 |
| Tapioca Starch + Water | 4.00 |
| Carbomer | 0.10 |
| Ammonium Acryldimethyltaurate/VP Copolymer | 0.50 |
| N-Acetylhydroxyproline | 1.00 |
| Carnitine | 2.00 |
| Perfume, Preservatives | q.s. |
| Water | ad 100 |

### Example 9: O/W Emulsion

| **Ingredient (INCI)** | **% by weight** |
|---|---|
| PEG-40 Stearate | 1.00 |
| Glyceryl Stearate | 2.50 |
| C12-15 Alkyl Benzoate | 1.00 |
| Octyldodecanol | 0.50 |
| Cetearyl Alcohol | 2.50 |
| Simmondsia Chinensis | 0.50 |
| Glycerin | 5.00 |
| Ethylhexyl Methoxycinnamate + BHT | 7.50 |
| Tapioca Starch + Water | 4.00 |
| Benzophenone-3 | 3.00 |
| Simmondsia Chinensis | 0.50 |
| Carbomer | 0.15 |
| Ammonium Acryldimethyltaurate/VP Copolymer | 0.50 |
| N-Acetylhydroxyproline | 1.00 |
| Carnitine | 2.00 |
| Perfume, Preservatives | q.s. |
| Water | ad 100 |

### Example 10: Hand Creams

| | **% by weight** | | |
|---|---|---|---|
| **Ingredient (INCI)** | **1** | **2** | **3** |
| Glyceryl Stearate | - | 2.5 | 1.5 |
| Stearic Acid | - | - | 2 |
| PEG-40 Stearate | - | 1 | - |
| Sorbitan Stearate | 2.5 | - | - |
| Trilaureth-4 Phosphate | 2 | - | - |
| Trisodium EDTA | - | 1 | - |
| Cera Microcristallina + Paraffinum Liquidum | 2.5 | - | 1 |
| Ethylhexyl Methoxycinnamate + BHT | - | 5 | - |
| Butyl Methoxydibenzoylmethane | - | 1 | - |
| Methylpropanediol | - | 3 | - |
| Glycerin | 3 | 3 | 8 |
| Ethylhexylglycerin | - | 0.5 | - |
| Isopropyl Palmitate | 2 | - | - |
| Caprylic/Capric Triglyceride | - | - | 1 |
| Octyldodecanol | 1.5 | - | - |
| Myristyl Myristate | - | 2 | - |
| C12-15 Alkyl Benzoate | - | 2 | 1 |
| Dicaprylyl Carbonate | - | 2.5 | - |
| Citric Acid, Sodium Citrate | 0.05 | - | - |
| Dimethicone | 1 | 2 | 0.75 |
| Powder Materials (z.B. TiO2, Starch, etc.) | - | 2 | - |
| Myristyl Alcohol | 3.5 | - | - |
| Cetearyl Alcohol | - | - | 3 |
| Cetyl Alcohol | 4.5 | 2 | - |
| Carbomer | 0.1 | 0.3 | 0.35 |
| Tocopheryl Acetate | - | - | 0.5 |
| Panthenol | 0.75 | - | 0.5 |
| Creatine | - | 0.2 | - |
| N-Acetylhydroxyproline | 0.2 | 0.5 | 1.0 |
| Preservative, Perfume, Base | q.s. | q.s. | q.s. |
| Water | ad 100 | ad 100 | ad 100 |

### Example 11

| | **1** | **2** |
|---|---|---|
| **Ingredient (INCI)** | **% by weight** | |
| Polyglyceryl-3-methyl glucose distearate | 2.00 | 2.00 |
| Stearyl alcohol | 2.00 | 1.00 |
| Sorbitan stearate | - | 1.00 |
| C₁₂₋₁₅ Alkyl benzoate | 3.00 | 3.00 |
| Butylene glycol dicaprylate/dicaprate | 2.00 | - |
| Cyclomethicone | 3.00 | 3.00 |
| Caprylic/capric triglyceride | 2.00 | 3.00 |
| Hydrogenated polydecene | 2.00 | 2.00 |
| Dimethylpolysiloxane (dimethicone) | 1.00 | 1.00 |
| Petrolatum | 1.00 | - |
| TiO₂ | 1.00 | 1.00 |
| Butyl methoxydibenzoylmethane | 2.00 | - |
| Bis-Ethylhexyloxyphenol methoxyphenyltriazine | - | 2.00 |
| Ethylhexyl cyanodiphenylacrylate (Octocrylene) | 5.00 | - |
| Ethylhexyltriazone | | 2.00 |
| Ethylhexyl methoxycinnamate | | 3.00 |
| N-Acetylhydroxyproline | 1.00 | 2.00 |
| Ubiquinone (Q10) | 0.10 | - |
| Allantoin | - | 0.10 |
| Phenoxyethanol | 0.40 | 0.40 |
| Iodopropynylbutyl carbamate | 0.05 | - |
| p-Hydroxybenzoic alkyl ester (paraben) | 0.20 | 0.20 |
| Xanthan gum | 0.10 | 0.10 |
| Carbomer | 0.10 | 0.10 |
| Butylene glycol | 3.00 | 3.00 |
| Aluminium starch octenylsuccinate | 2.00 | |
| Nylon 12 | - | 3.00 |
| Additives (talc, BHT, EDTA) | 0.50 | 0.50 |
| Perfume | q.s. | q.s. |
| Water | ad 100 | ad 100 |

### Example 12:

| | **1** | **2** |
|---|---|---|
| **Ingredient (INCI)** | **% by weight** | |
| Polyglyceryl-3-methyl glucose distearate | 3.00 | 3.00 |
| Sorbitan stearate | 1.00 | 1.00 |
| C₁₂₋₁₅ Alkyl benzoate | 2.00 | 2.00 |
| Ethylhexyl coconut fatty acid ester | 5.00 | 5.00 |
| Octamethyltetrasiloxane (cyclomethicone) | 5.00 | 5.00 |
| Polydecene | 1.00 | 1.00 |
| Bis-ethylhexyloxyphenol-methoxyphenyltriazine | | 3.00 |
| Ethylhexylmethoxycinnamate | 4.00 | 5.00 |
| Ethylhexyltriazone | 1.00 | - |
| Diethylhexylbutamido-triazone | - | 1.00 |
| 2,2'-(1,4-Phenylene)-bis(1H-benzimidazole-4,6-disulfonic acid) | 2.00 | - |
| N-Acetylhydroxyproline | 1.00 | 1.50 |
| Ubiquinone (Q10) | - | 0.10 |
| Tocopherol | 0.10 | 0.10 |
| EDTA | 0.20 | 0.20 |
| para-Hydroxybenzoic alkyl ester (paraben) | 0.30 | 0.20 |
| Methylpropanediol | 3.00 | 3.00 |
| Ethanol, denaturated | 2.00 | 2.00 |
| Sodium ascorbylphosphate | 0.10 | - |
| Xanthan gum | 0.20 | 0.20 |
| Carbomer | 0.10 | 0.10 |
| Glycerin | 5.00 | 7.00 |
| Sodium starch octenylsuccinate | 2.00 | - |
| Filler/additives (distarch phosphate, talc) | 0.2 | 2.00 |
| Perfume | q.s. | q.s. |
| Water | ad 100 | ad 100 |

### Example 13:

| | **1** | **2** |
|---|---|---|
| **Ingredient (INCI)** | **% by weight** | |
| Glyceryl stearate citrate | 2.50 | 1.50 |
| Cetyl stearyl alcohol | 1.00 | 4.00 |
| Caprylic/capric triglyceride | 2.00 | 1.00 |
| Dicaprylcarbonate | 2.00 | 2.00 |
| Octyldodecanol | 2.00 | - |
| Cyclomethicone | 2.00 | 1.00 |
| C₁₂₋₁₅ Alkylbenzoate | 2.00 | 3.00 |
| TiO₂ | 2.00 | - |
| Butyl methoxydibenzoylmethane | - | 2.00 |
| Ethylhexyl methoxycinnamate | 3.00 | 5.00 |
| Ethylhexyltriazone | - | 2.00 |
| Carbomer | 0.10 | 0.3 |
| Acrylic acid/C₁₀₋₃₀ alkylmethacrylate-copolymer | 0.3 | - |
| Dimethicone | 0.75 | 0.75 |
| Jojoba oil | - | 1.00 |
| Tocopheryl acetate | 0.50 | 0.50 |
| Glycerin | 7.00 | 10.00 |
| Ethanol | - | 1.00 |
| N-Acetylhydroxyproline | 1.00 | 0.50 |
| Fillers/additives (distarch phosphate, BHT, talc, aluminum starch octenylsuccinate, cyclodextrin, EDTA) | 2.00 | 1.00 |
| Perfume | q.s. | q.s. |
| Preservatives | q.s. | q.s. |
| Water | ad 100 | ad 100 |

### Example 14: O/W Emulsions

| | **1** | **2** | **3** |
|---|---|---|---|
| **Ingredient (INCI)** | **% by weight** | | |
| Glyceryl stearate citrate | 2.00 | 2.00 | 2.00 |
| Cetyl alcohol | 1.00 | 1.00 | 1.00 |
| Cyclomethicone | 3.00 | 1.00 | 5.00 |
| C₁₂₋₁₅ Alkyl benzoate | 2.00 | 3.00 | 1.00 |
| Caprylic/capric triglyceride | 2.00 | 1.00 | 2.00 |
| Dicaprylcarbonate | 2.00 | 3.00 | - |
| Macadamia oil | - | 0.50 | - |
| Paraffinium liquidum | 1.00 | 1.00 | 1.00 |
| Methylene-bis-6-(2H-benzotriazolyl)-4-(tetramethylbutyl)-1,1,3,3-phenol | 2.00 | - | - |
| Bis-Ethylhexyloxyphenol-methoxyphenyltriazine | - | 2.00 | 3.00 |
| Ethylhexyl methoxycinnamate | 5.00 | 7.00 | 5.00 |
| Ethylhexyltriazone | - | 2.00 | - |
| N-Acetylhydroxyproline | 0.25 | 0.50 | 0.75 |
| Glycerin | 5.00 | 7.00 | 9.00 |
| Serine | 0.10 | - | - |
| Tocopherol acetate | 1.00 | 0.50 | 0.50 |
| Aluminum starch octenylsuccinate | 2.00 | 1.00 | - |
| Carbomer | 0.20 | 0.10 | |
| C10-30 Alkyl/Acrylates Crosspolymer | - | 0.30 | 0.20 |
| Xanthan gum | 0.10 | | 0.10 |
| Perfume, preservatives, colorants, antioxidants, etc. | q.s. | q.s. | q.s. |
| Water | ad 100 | ad 100 | ad 100 |

| | | | |
|---|---|---|---|
| pH value adjusted to 6.0 | | | |

### Example 15: O/W Emulsion

| **Ingredient (INCI)** | **% by weight** |
|---|---|
| PEG-40 Stearate | 1.00 |
| Glyceryl Stearate | 2.50 |
| C12-15 Alkyl Benzoate | 1.00 |
| Octyldodecanol | 0.50 |
| Cetylstearyl alcohol | 2.00 |
| Simmondsia Chinensis | 0.50 |
| Glycerin | 5.00 |
| Butyl methoxydibenzoylmethane | 2.00 |
| Diethylamino hydroxybenzoyl hexyl benzoate | 2.00 |
| Ethylhexyl Methoxycinnamate + BHT | 5.00 |
| Tapioca Starch + Water | 4.00 |
| Carbomer | 0.10 |
| Ammonium Acryldimethyltaurate/VP Copolymer | 0.50 |
| N-Acetylhydroxyproline | 1.00 |
| L-Carnitine | 0.50 |
| Perfume, Preservatives | q.s. |
| Water | ad 100 |

### Example 16: O/W Emulsion

| **Ingredient (INCI)** | **% by weight** |
|---|---|
| PEG-40 Stearate | 1.00 |
| Glyceryl Stearate | 2.50 |
| C₁₂₋₁₅ Alkyl Benzoate | 1.00 |
| Octyldodecanol | 0.50 |
| Cetylstearyl alcohol | 2.00 |
| Bis-Ethylhexyloxyphenol-methoxyphenyltriazine | 2.00 |
| Ethylhexylmethoxycinnamate | 4.00 |
| Phenylbenzimidazole sulfonic acid | 1.00 |
| Simmondsia Chinensis | 0.50 |
| Glycerin | 5.00 |
| Ethylhexyl Methoxycinnamate + BHT | 7.50 |
| Tapioca Starch + Water | 4.00 |
| Creatine | 0.50 |
| Simmondsia Chinensis | 0.50 |
| Carbomer | 0.15 |
| Polyacrylate-3 | 0.30 |
| N-Acetylhydroxyproline | 1.00 |
| Aloe Vera | 0.10 |
| Perfume, Preservatives | q.s. |
| Water | ad 100 |

| | |
|---|---|
| pH value adjusted to 7.0 | |

### Example 17: O/W Emulsions

| | **% by weight** | | |
|---|---|---|---|
| **Ingredient (INCI)** | **1** | **2** | **3** |
| Glyceryl Stearate | - | 2.5 | 1.5 |
| Stearic Acid | - | - | 2 |
| PEG-40 Stearate | - | 1 | - |
| Sorbitan Stearate | 2.5 | - | - |
| Trilaureth-4 Phosphate | 2 | - | - |
| Trisodium EDTA | - | 0.4 | 0.2 |
| Cera Microcristallina + Paraffinum Liquidum | 2.5 | - | 1 |
| Ethylhexyl Methoxycinnamate + BHT | 5.00 | 5 | 5.00 |
| Butyl Methoxydibenzoylmethane | - | 1 | - |
| Benzophenone-3 | 3.00 | | |
| Bis-Ethylhexyloxyphenol-methoxyphenyltriazine | | | 2.00 |
| Methylpropanediol | - | 3 | - |
| Glycerin | 3 | 3 | 8 |
| Ethylhexylglycerin | - | 0.5 | - |
| Isopropyl Palmitate | 2 | - | - |
| Caprylic/Capric Triglyceride | - | - | 1 |
| Octyldodecanol | 1.5 | - | - |
| Myristyl Myristate | - | 2 | - |
| C₁₂₋₁₅ Alkyl Benzoate | - | 2 | 1 |
| Dicaprylyl Carbonate | - | 2.5 | - |
| Citric Acid, Sodium Citrate | 0.05 | - | - |
| Dimethicone | 1 | 2 | 0.75 |
| Powder Materials (z.B. TiO₂, distarch phosphate, nylon 12, talc etc.) | - | 2 | 2 |
| Myristyl alcohol | 3.5 | - | - |
| Cetylstearly alcohol | - | - | 3 |
| Cetyl alcohol | 4.5 | 2 | - |
| Carbomer | 0.1 | 0.1 | 0.1 |
| Polyacrylate-3 (methacrylic acid/methylmethacrylate/methylstyrene/isopropylisocyanate and PEG-40 behenate) | - | - | 0.2 |
| Carageenan | - | 0.2 | - |
| Tocopheryl Acetate | - | - | 0.5 |
| Panthenol | 0.5 | - | 0.5 |
| Creatine | - | 0.5 | - |
| Vitamin A palmitate | | | 0.1 |
| Glycyrrhiza inflata (licochalcone A) | | 0.1 | |
| N-Acetylhydroxyproline | 0.2 | 0.5 | 1.0 |
| Preservative, Perfume, Base | q.s. | q.s. | q.s. |
| Water | ad 100 | ad 100 | ad 100 |

### Example 18: O/W Cream

| **Ingredient** (**INCI**) | **% by weight** |
|---|---|
| Cetearyl glucoside | 3.00 |
| Myristyl myristate | 1.00 |
| Cetyl alcohol | 3.00 |
| C₁₂₋₁₅ Alkylbenzoate | 2.00 |
| Caprylic/Capric Triglyceride | 3.00 |
| Hydrogenated Polydecene | 1.00 |
| Dicaprylyl carbonate | 3.00 |
| Ethylhexyl methoxycinnamate | 5.00 |
| Ethylhexyl cyanodiphenylacrylate (Octocrylene) | 3.00 |
| Butyl methoxydibenzoylmethane | 2.00 |
| N-Acetylhydroxyproline | 0.30 |
| Retinyl palmitate | 0.10 |
| Tocopheryl acetate | 1.00 |
| Trisodium EDTA | 0.20 |
| p-Alkyl Hydroxybenzoate (Paraben) | 0.30 |
| 1,3-Dimethylol-5,5-dimethyl-hydantoin(DMDM Hydantoin) | 0.1 |
| Butyleneglycol | 3.00 |
| Xanthan Gum | 0.20 |
| Aluminum starch octenylsuccinate | 1.00 |
| Glycerin | 6.00 |
| Additives (talc, BHT, colorants) | 1.00 |
| Perfume | q.s. |
| Water | ad 100 |

### Example 19: W/O Cream

| **Ingredient (INCI)** | **% by weight** |
|---|---|
| Polyglyceryl-3 -diisostearate | 5.0 |
| Polyglyceryl-2-dipolyhydroxystearate | 2.5 |
| Cetylstearyl alcohol | 2 |
| C₁₂₋₁₅ Alkylbenzoate | 4 |
| Caprylic/Capric Triglyceride | 2 |
| Octyldodecanol | 1 |
| Paraffinum liquidum | 4 |
| Octamethyltetrasiloxane (Cyclomethicone) | 3 |
| Lactic acid | 1 |
| Citric acid, sodium salt | 0.5 |
| Butylmethoxydibenzoylmethane | 1 |
| Ethylhexyltriazone | 1 |
| Ethylhexyl methoxycinnamate | 5 |
| N-Acetylhydroxyproline | 0.5 |
| Urea | 2 |
| p-Alkyl hydroxybenzoate (Paraben) | 0.1 |
| Glycerin | 7 |
| Fillers (EDTA, BHT) | 0.3 |
| Perfume | q.s. |
| Water | ad 100 |

### Example 20: O/W Cream

| **Ingredient (INCI)** | **% by weight** |
|---|---|
| Glyceryl stearate SE (self-emulsifying) | 5 |
| Stearyl alcohol | 2 |
| Shea butter | 1 |
| C₁₂₋₁₅ Alkylbenzoate | 3 |
| Caprylic/Capric Triglyceride | 2 |
| Petrolatum | 1 |
| Sunflower oil | 0.5 |
| Dicaprylyl carbonate | 3 |
| Ethylhexyl methoxycinnamate | 3 |
| Ethylhexyltriazone | 1 |
| Bis-ethylhexyloxyphenol-methoxyphenyltriazine | 2 |
| N-Acetylhydroxyproline | 0.50 |
| Citric acid, sodium salt | 0.1 |
| Phenoxyethanol | 0.4 |
| p-Alkyl hydroxybenzoate (Paraben) | 0.3 |
| Hexamidine diisethionate | 0.04 |
| Soy extract | 0.5 |
| Tocopheryl acetate | 0.5 |
| EDTA | 0.2 |
| Carbomer | 0.3 |
| Distarch Phosphate | 2 |
| Glycerin | 8 |
| Additives (SiO₂, BHT) | 0.2 |
| Perfume | q.s. |
| Water | ad 100 |

### Example 21: W/O-Emulsions

| | **1** | **2** | **3** | **4** | **5** |
|---|---|---|---|---|---|
| **Ingredient (INCI)** | **% by weight** | | | | |
| Triglycerin diisostearate | 1.0 | 0.5 | 0.5 | 2.0 | -- |
| Diglycerin dipolyhydroxystearate | 1.0 | 1.5 | 1.75 | 3.0 | -- |
| Paraffin oil | 12.5 | 10.0 | 8.0 | 5.0 | 7.5 |
| Petrolatum | 8.0 | 6.0 | 5.0 | 12.0 | 2.5 |
| Hydrogenated cocoglycerides | 2.0 | 1.0 | 2.5 | 5.0 | 0.25 |
| Decyl oleate | 0.5 | 0.75 | 1.0 | 2.0 | 0.25 |
| Octyldodecanol | 0.5 | 1.0 | 0.75 | 3.0 | 0.25 |
| Aluminum stearate | 0.4 | 0.3 | 0.6 | 1.0 | 0.05 |
| Dicaprylyl carbonate | 0.1 | 0.05 | 0.15 | 0.5 | 1.0 |
| Hydrogenated castor oil | 0.5 | 0.75 | 1.0 | 2.5 | 5.0 |
| Microcrystalline cellulose | 0.5 | 1.0 | --- | 0.25 | 0.1 |
| Magnesium sulfate | 0.5 | 0.6 | 0.5 | 0.7 | 1.0 |
| Glycerin | 3.0 | 5.0 | 10.0 | 15.0 | 1.5 |
| Citric acid | 0.2 | 0.1 | 0.2 | 0.3 | 1.0 |
| Sodium citrate | 0.2 | 0.05 | 0.4 | 0.3 | 2.0 |
| Perfume | q.s. | q.s. | q.s. | q.s. | q.s. |
| Ethanol | 2.0 | --- | 5.0 | --- | --- |
| Caprylic/Capric triglycerides | 2.0 | 2.5 | 3.0 | 5.0 | 0.5 |
| Potassium sorbate | 0.04 | 0.15 | 0.05 | 0.03 | 0.4 |
| Benzyl alcohol | 0.3 | 0.4 | 0.25 | 0.15 | --- |
| N-Acetylhydroxyproline | 0.1 | 0.25 | 2.5 | 5.0 | 12.5 |
| Wool wax alcohol | 1.5 | 0.3 | 0.5 | 1.0 | 5.0 |
| Water | ad 100 | ad 100 | ad 100 | ad 100 | ad 100 |

### Example 22: W/O-Emulsions

| | **1** | **2** | **3** | **4** | **5** |
|---|---|---|---|---|---|
| **Ingredient (INCI)** | **% by weight** | | | | |
| PEG-30 Dipolyhydroxystearate | --- | 0.5 | 0.25 | --- | 5.0 |
| Wool wax alcohol | 1.0 | 1.5 | 1.75 | 3.0 | -- |
| Paraffin oil | 12.5 | 10.0 | 8.0 | 5.0 | 7.5 |
| Petrolatum | 8.0 | 6.0 | 5.0 | 12.0 | 2.5 |
| Hydrogenated cocoglycerides | 2.0 | 1.0 | 2.5 | 5.0 | 0.25 |
| Hydrogenated polyisobutene | 0.5 | 0.75 | 1.0 | 2.0 | 0.25 |
| Octyldodecanol | 0.5 | 1.0 | 0.75 | 3.0 | 0.25 |
| Aluminum stearate | 0.4 | 0.3 | 0.6 | 1.0 | 0.05 |
| Dicaprylyl carbonate | 0.1 | 0.05 | 0.15 | 0.5 | 1.0 |
| Hydrogenated castor oil | 0.5 | 0.75 | 1.0 | 2.5 | 5.0 |
| Table paraffin | 0.5 | 1.0 | 0.75 | 0.25 | 0.1 |
| Magnesium sulfate | 0.5 | 0.6 | 0.5 | 0.7 | 1.0 |
| Glycerin | 3.0 | 5.0 | 10.0 | 15.0 | 1.5 |
| Citric acid | 0.2 | 0.1 | 0.2 | 0.3 | 1.0 |
| N-Acetylhydroxyproline | 0.1 | 0.25 | 2.5 | 7.5 | 20.5 |
| Perfume | q.s. | q.s. | q.s. | q.s. | q.s. |
| 1,3-Butyleneglycol | 2.0 | --- | 5.0 | --- | --- |
| Caprylic/capric triglyceride | 2.0 | 2.5 | 3.0 | 5.0 | 0.5 |
| Sodium dehydracetate | --- | --- | 0.05 | --- | --- |
| Potassium sorbate | 0.3 | 0.4 | 0.25 | 0.15 | --- |
| Talc | --- | --- | 0.05 | --- | 0.1 |
| Water | ad 100 | ad 100 | ad 100 | ad 100 | ad 100 |

### Example 23: W/S-Emulsions

| | **1** | **2** | **3** | **4** | **5** |
|---|---|---|---|---|---|
| **Ingredient (INCI)** | **% by weight** | | | | |
| Cetyl PEG/PPG-10/1 Dimethicone | 1.0 | --- | --- | 3.0 | 5.0 |
| Cylomethicone + PEG/PPG-18/18 Dimethicone (90:10) | 10.0 | 12.5 | 25 | -- | -- |
| Petrolatum | 12.5 | 15 | 28.0 | 25.0 | 17.5 |
| Dimethicone | 5.0 | 13.0 | 5.0 | 12.0 | 15.0 |
| Hydrogenated polyisobutene | 0.5 | 0.75 | 1.0 | 2.0 | 0.25 |
| Mineral oil | 0.5 | 1.0 | 0.75 | 3.0 | 0.25 |
| Panthenol | 0.5 | 1.0 | 0.75 | 0.25 | 0.1 |
| Sodium chloride | 2.0 | 0.6 | 2.5 | 0.7 | 1.0 |
| Glycerin | 3.0 | 5.0 | 10.0 | 15.0 | 1.5 |
| Citric acid | 0.2 | 0.1 | 0.2 | 0.3 | 1.0 |
| Sodium citrate | 1.0 | 0.1 | 0.4 | 0.9 | 2.5 |
| N-Acetylhydroxyproline | 0.1 | 0.25 | 2.5 | 5.0 | 10 |
| Perfume | q.s. | q.s. | q.s. | q.s. | q.s. |
| Potassium sorbate | 0.4 | 0.1 | 0.05 | 0.3 | 0.4 |
| Wool wax | 1.0 | 0.5 | 2.5 | 0.25 | 5.5 |
| Cetyldimethicone | 0.5 | --- | 0.7 | --- | --- |
| Benzyl alcohol | --- | --- | 0.05 | --- | 0.1 |
| Water | ad 100 | ad 100 | ad 100 | ad 100 | ad 100 |

### Example 24: W/S-Emulsions

| | **1** | **2** | **3** | **4** | **5** |
|---|---|---|---|---|---|
| **Ingredient (INCI)** | **% by weight** | | | | |
| Cetyl PEG/PPG-10/1 Dimethicone | 1.0 | --- | -- | 3.0 | 5.0 |
| Cylomethicone + PEG/PPG-18/18 Dimethicone (90:10) | 10.0 | 12.5 | 25 | -- | -- |
| Cyclomethicone | 12.5 | 15 | 28.0 | 25.0 | 17.5 |
| Dimethicone | 5.0 | 13.0 | 5.0 | 12.0 | 15.0 |
| Microwax | 0.5 | 0.75 | 1.0 | 2.0 | 0.25 |
| Octyldodecanol | 0.5 | 1.0 | 0.75 | 3.0 | 0.25 |
| Panthenol | 0.5 | 1.0 | 0.75 | 0.25 | 0.1 |
| Sodium chloride | 2.0 | 0.6 | 2.5 | 0.7 | 1.0 |
| Glycerin | 3.0 | 5.0 | 10.0 | 15.0 | 1.5 |
| Lactic acid | 0.2 | 0.1 | 0.2 | --- | --- |
| Sodium lactate | 0.2 | 1.0 | 0.05 | --- | --- |
| Perfume | q.s. | q.s. | q.s. | q.s. | q.s. |
| N-Acetylhydroxyproline | 0.1 | 0.25 | 2.5 | 5.0 | 7.5 |
| Wool wax alcohol | 1.0 | 1.5 | 1.5 | 0.25 | 0.1 |
| Stearyl dimethicone | 0.5 | --- | 0.7 | --- | --- |
| Dehydracetic acid | --- | --- | 0.05 | --- | 0.1 |
| Modified Starch | --- | 2.5 | --- | 0.15 | -- |
| Water | ad 100 | ad 100 | ad 100 | ad 100 | ad 100 |

### Example 25: W/O-Emulsions

| | **1** | **2** | **3** | **4** | **5** |
|---|---|---|---|---|---|
| **Ingredient (INCI)** | **% by weight** | | | | |
| PEG-22 Dodecyl Glycol Copolymer | 5.0 | 1.5 | 0.25 | --- | 3.0 |
| PEG-45 Dodecyl Glycol Polymer | 1.0 | 1.5 | 1.75 | 3.0 | -- |
| Paraffin oil | 12.5 | 10.0 | 8.0 | 5.0 | 17.5 |
| Isopropyl stearate | 8.0 | 6.0 | 5.0 | 12.0 | 2.5 |
| Table paraffin | 2.0 | 1.0 | 2.5 | 5.0 | 0.25 |
| Evening primrose oil | 0.5 | 0.75 | 1.0 | 2.0 | 0.25 |
| Petrolatum | 0.5 | 1.0 | 0.75 | 3.0 | 0.25 |
| Aluminum stearate | 0.4 | 0.3 | 0.6 | 1.0 | 0.05 |
| Dicaprylyl carbonate | 0.1 | 0.05 | 0.15 | 0.5 | 1.0 |
| Hydrogenated castor oil | 0.5 | 0.75 | 1.0 | 2.5 | 5.0 |
| Wool wax | 0.5 | 1.0 | 7.5 | 0.25 | 0.1 |
| Magnesium sulfate | 0.5 | 0.6 | 0.5 | 0.7 | 1.0 |
| N-Acetylhydroxyproline | 0.15 | 2.5 | 3.5 | 9.0 | 12.5 |
| Glycerin | 3.0 | 5.0 | 10.0 | 15.0 | 1.5 |
| Sodium citrate | 0.2 | 0.1 | --- | --- | --- |
| Citric acid | 0.2 | 0.1 | --- | --- | --- |
| Perfume | q.s. | q.s. | q.s. | q.s. | q.s. |
| 1,3-Butylene glycol | 2.0 | --- | 5.0 | --- | --- |
| Caprylic/capric triglycerides | 2.0 | 2.5 | 3.0 | 5.0 | 0.5 |
| Potassium sorbate | 0.4 | 0.15 | 0.05 | 0.3 | 0.4 |
| Benzyl alcohol | --- | --- | 0.05 | --- | 0.1 |
| Water | ad 100 | ad 100 | ad 100 | ad 100 | ad 100 |

### Example 26: W/O-Emulsions

| | **1** | **2** | **3** | **4** | **5** |
|---|---|---|---|---|---|
| **Ingredient (INCI)** | **% by weight** | | | | |
| Polyglyceryl-2-dipolyhydroxystearate | 3.0 | --- | 0.25 | --- | 3.0 |
| Polyglyceryl-3 -diisostearate | 1.0 | 3.5 | 1.75 | 2.5 | --- |
| PEG-40 sorbitan isostearate | --- | 2.5 | 0.5 | 3.5 | 3.0 |
| Paraffin oil | 12.5 | 10.0 | 8.0 | 5.0 | 17.5 |
| Microwax | 2.5 | 1.5 | 5.0 | 1.2 | 0.25 |
| Hydrogenated cocoglycerides | 2.0 | 1.0 | 2.5 | 5.0 | 0.25 |
| Isopropyl palmitate | 0.5 | 1.0 | 0.75 | 3.0 | 0.25 |
| Dicaprylyl carbonate | 0.1 | 0.05 | 0.15 | 0.5 | 1.0 |
| Hydrogenated castor oil | 0.5 | 0.75 | 1.0 | 2.5 | 5.0 |
| Wool wax alcohol | 0.5 | 1.0 | 0.75 | 0.25 | 0.1 |
| N-Acetylhydroxyproline | 0.1 | 0.25 | 2.5 | 5.0 | 12.5 |
| Magnesium sulfate | 0.5 | 0.6 | 0.5 | 0.7 | 1.0 |
| Glycerin | 3.0 | 5.0 | 10.0 | 15.0 | 1.5 |
| Citric acid | 0.2 | 0.1 | 0.1 | 0.3 | 1.0 |
| Sodium citrate | 0.2 | 0.3 | 0.2 | 1.5 | 0.8 |
| Perfume | q.s. | q.s. | q.s. | q.s. | q.s. |
| Caprylic/capric triglycerides | 2.0 | 2.5 | 3.0 | 5.0 | 0.5 |
| Potassium sorbate | 0.24 | 0.15 | 0.05 | 0.3 | 0.4 |
| Water | ad 100 | ad 100 | ad 100 | ad 100 | ad 100 |

### Example 27: S/W-Emulsions

| | **1** | **2** | **3** | **4** | **5** |
|---|---|---|---|---|---|
| **Ingredient (INCI)** | **% by weight** | | | | |
| Dimethicone copolyol, Caprylic/Capric triglycerides | 1.0 | 2.0 | 8.0 | 3.0 | 5.0 |
| Cyclomethicone | 12.5 | 15 | 25.0 | 10.0 | 7.5 |
| Dimethicone | 5.0 | 15.0 | 5.0 | 12.0 | 15.0 |
| Mineral oil | 0.5 | 0.75 | 1.0 | 2.0 | 0.25 |
| Phenyl trimethicone | 0.5 | 1.0 | 0.75 | 3.0 | 0.25 |
| Glycerin | 5.0 | 7.5 | 10.0 | 3.0 | 1.0 |
| N-Acetylhydroxyproline | 0.1 | 0.25 | 2.5 | 5.0 | 12.5 |
| Xanthan Gum | --- | 0.1 | -- | 0.25 | 1.0 |
| Panthenol | 0.5 | 1.0 | 0.75 | 0.25 | 0.1 |
| Perfume | q.s. | q.s. | q.s. | q.s. | q.s. |
| Methylparaben | 0.4 | 0.1 | 0.05 | 0.3 | 0.4 |
| Propylparaben | 0.3 | 0.4 | 0.25 | 0.15 | --- |
| Iodopropynyl butylcarbamate | --- | --- | 0.05 | --- | 0.1 |
| Wool wax alcohol | 0.5 | 1.5 | 1.0 | 0.75 | 0.25 |
| Water | ad 100 | ad 100 | ad 100 | ad 100 | ad 100 |

### Example 28: O/W-Emulsions

| | **1** | **2** | **3** | **4** | **5** |
|---|---|---|---|---|---|
| **Ingredient (INCI)** | **% by weight** | | | | |
| Glyceryl stearate | 1.0 | --- | --- | 0.5 | 0.25 |
| Polyethylenglycol(40) stearate | 10.0 | --- | 5 | -- | -- |
| Triglycerin methylglucose distearate | --- | 5.5 | --- | --- | 2.5 |
| Sorbitan stearate | --- | 1.5 | 3 | --- | --- |
| Mineral oil, low viscosity | 2.5 | 15 | 8.0 | 5.0 | 7.5 |
| Dimethicone | 5.0 | 3.0 | 5.0 | 2.0 | 5.0 |
| Behenyl alcohol | 1 | --- | 2 | 1 | --- |
| N-Acetylhydroxyproline | 0.1 | 0.25 | 2.5 | 7.5 | 2.5 |
| Stearyl alcohol | --- | 1 | --- | 1 | --- |
| Wool wax alcohol | 5 | 10 | 15 | 20 | 2.5 |
| Cetylstearyl alcohol | --- | --- | 1 | 1 | --- |
| Hydrogenated Polyisobutene | 0.5 | 0.75 | 1.0 | 2.0 | 0.25 |
| Octyldodecanol | 0.5 | 1.0 | 0.75 | 3.0 | 0.25 |
| Perfume | q.s. | q.s. | q.s. | q.s. | q.s. |
| Methylparaben | 0.4 | 0.1 | 0.05 | 0.3 | 0.4 |
| Propylparaben | 0.3 | 0.4 | 0.25 | 0.15 | --- |
| Iodopropynyl butylcarbamate | --- | --- | 0.05 | --- | 0.1 |
| Glycerin | 5 | 10 | 3 | 15 | 7.5 |
| Modified starch | --- | 2.5 | --- | 0.15 | -- |
| Water | ad 100 | ad 100 | ad 100 | ad 100 | ad 100 |

### Example 29: O/W-Emulsions

| | **1** | **2** | **3** | **4** | **5** |
|---|---|---|---|---|---|
| **Ingredient (INCI)** | **% by weight** | | | | |
| Polyethylenglycol(21) stearylether | 1 | -- | 2.5 | 2 | 1.5 |
| Polyethylenglycol(2) stearylether | 1 | -- | 5.5 | 3 | 7.5 |
| Cetearylglucoside | --- | 8 | --- | --- | --- |
| Behenyl alcohol | 3 | 2 | --- | 1 | --- |
| Stearyl alcohol | 3 | 2 | --- | 2 | --- |
| Cetylstearyl alcohol | 3 | 4 | --- | --- | 2 |
| Mineral oil, high viscosity | 0.5 | 0.75 | 1:0 | 2.0 | 0.25 |
| Petrolatum | 5 | 3.5 | 2.5 | 1.0 | 7.5 |
| N-Acetylhydroxyproline | 1.25 | 0.25 | 2.5 | 5.0 | 20.5 |
| Octyldodecanol | 0.5 | 1.0 | 0.75 | 3.0 | 0.25 |
| Glycerin | 5 | 10 | 15 | 3 | 7.5 |
| Panthenol | 0.5 | 1.0 | 0.75 | 0.25 | 0.1 |
| Perfume | q.s. | q.s. | q.s. | q.s. | q.s. |
| Methylparaben | 0.4 | 0.1 | 0.05 | 0.3 | 0.4 |
| Propylparaben | 0.3 | 0.4 | 0.25 | 0.15 | --- |
| Iodopropynyl butylcarbamat | --- | --- | 0.05 | --- | 0.1 |
| Modified starch | 0.5 | --- | --- | 0.15 | -- |
| Water | ad 100 | ad 100 | ad 100 | ad 100 | ad 100 |

### Example 30: W/O-Emulsions

| | **1** | **2** | **3** | **4** | **5** |
|---|---|---|---|---|---|
| **Ingredient (INCI)** | **% by weight** | | | | |
| Glyceryl stearate citrate | 1.0 | 0.5 | 0.1 | 0.5 | 0.3 |
| Polyethylenglycol(20) cetearylether | 10.0 | 1.0 | 5 | --- | --- |
| Triglycerin methylglucose distearate | --- | --- | --- | --- | 2.5 |
| Ethylbutylacetyl aminopropionate | 5 | --- | 15 | 20 | --- |
| N-Acetylhydroxyproline | 0.1 | 0.5 | 2.5 | 5.0 | 2.5 |
| Microwax | 1.0 | 1.5 | 0.5 | 0.75 | 2.25 |
| Dimethicone | 0.5 | 3.0 | 0.75 | 1.5 | 0.2 |
| Behenyl alcohol | 1 | --- | 2 | 1 | 0.2 |
| Dicaprylyl carbonate | 3 | 5 | 10 | 15 | 5 |
| Stearyl alcohol | --- | --- | --- | 1 | 0.2 |
| Cetylstearyl alcohol | --- | --- | 1 | 1 | 0.2 |
| Tocopherol | 0.5 | 0.5 | 0.75 | 0.25 | 0.1 |
| Octyldodecanol | 0.5 | --- | 0.75 | 3.0 | 0.25 |
| Panthenol | 0.5 | --- | 0.75 | 0.25 | 0.1 |
| Carbomer | 0.05 | 0.35 | 0.15 | 0.1 | --- |
| Perfume | q.s. | q.s. | q.s. | q.s. | q.s. |
| Caprylic/capric triglycerides | 1 | 5 | 3 | 5 | 10 |
| Methylparaben | 0.4 | 0.3 | 0.05 | 0.3 | 0.4 |
| Propylparaben | 0.3 | --- | 0.25 | 0.15 | --- |
| Iodopropynyl butylcarbamat | --- | --- | 0.05 | --- | 0.1 |
| Phenoxyethanol | --- | 0.5 | --- | 0.15 | --- |
| Sorbitol | 10 | --- | -- | 5 | --- |
| Butylene glycol | --- | --- | --- | 5 | 10 |
| Propylene glycol | --- | --- | 10 | 5 | --- |
| Glycerin | --- | 7.5 | --- | --- | --- |
| Water | ad 100 | ad 100 | ad 100 | ad 100 | ad 100 |

### Example 31: Cationic O/W-Emulsions

| | **1** | **2** | **3** | **4** | **5** |
|---|---|---|---|---|---|
| **Ingredient (INCI)** | **% by weight** | | | | |
| | | | | | |
| Distearyl dimmoniumchloride | 1 | 2 | 3.5 | 4 | 5 |
| Cetylstearyl alcohol | 1 | --- | --- | 0.5 | --- |
| Cetyl alcohol | 1 | 2.5 | 2 | --- | 0.5 |
| Dimethylpolysiloxane | 1 | 1 | 1 | 3 | 5 |
| Evening primrose oil | 2 | --- | --- | 7.5 | --- |
| Table paraffin | 1 | 2.5 | 1 | --- | --- |
| Mineral oil, low viscosity | 2.5 | --- | 1 | 0.25 | 3.75 |
| Mineral oil, high viscosity | 3 | 5 | 6 | --- | --- |
| Petrolatum | --- | 4.5 | 4 | --- | --- |
| Iodopropynyl butylcarbamate | --- | 0.2 | 0.2 | --- | --- |
| Polyhexamethylenbiguanide hydrochloride | 0.5 | 1 | 1 | 2 | 3 |
| N-Acetylhydroxyproline | 0.5 | 1 | 1 | 12 | 3.5 |
| Glycerin | 10 | 15 | 12 | 25 | 8 |
| Behenyl alcohol | 1 | --- | --- | --- | 1 |
| Stearyl alcohol | 1 | 1 | --- | --- | 1 |
| Methylparaben | 0.1 | --- | --- | 0.1 | 0.4 |
| Benzyl alcohol | --- | 1 | --- | 0.5 | --- |
| Hydrogenated cocoglycerides | 2 | --- | --- | 1 | --- |
| Shea butter | --- | 2 | --- | 1 | --- |
| Butylenglycol dicaprylate/dicaprate | 1 | --- | --- | --- | 5 |
| Caprylic/capric triglycerides | --- | 4 | 3 | --- | 1 |
| Ethylhexyl coco fatty acid esters | 3 | --- | --- | 5 | --- |
| Octyldodecanol | --- | --- | 3 | --- | --- |
| Octamethyltetrasiloxane | --- | 1 | --- | 1 | 3 |
| Oatmeal | --- | 1.5 | 1 | 1 | --- |
| EDTA | 0.5 | --- | --- | 1 | --- |
| BHT | 0.03 | --- | --- | 0.05 | --- |
| Dicaprylyl carbonate | --- | 5 | --- | --- | 2 |
| Perfume | q.s. | q.s. | q.s. | q.s. | q.s. |
| Water | Ad 100 | Ad 100 | Ad 100 | Ad 100 | Ad 100 |

### Example 32: Gels

| | **1** | **2** | **3** | **4** | **5** |
|---|---|---|---|---|---|
| **Ingredient (INCI)** | **% by weight** | | | | |
| Sorbitan Stearate | 1 | 0.5 | 1 | 1.2 | 1.3 |
| Cetearyl alcohol + PEG-40 castor oil + sodium cetearyl sulfate | 2.5 | 3 | 3.2 | 3.5 | 4 |
| Phenoxyethanol | 0.3 | 0.8 | 0.4 | 0.6 | 0.4 |
| Methylparaben | 0.1 | 0.3 | 0.2 | 0.3 | 0.2 |
| Natriumlactat | 2 | 2 | 1.5 | - | - |
| Urea | 5 | - | - | - | - |
| Glycerin | 3 | 7.5 | 5 | 7.5 | 5 |
| Serine + Glycine+ Alanine + Lecithin + Disodium phosphate + Potassium phosphate | 2 | 0.1 | 0.25 | 1 | 2 |
| N-Acetylhydroxyproline | 2 | 2.2 | 1.1 | 0.5 | 1.1 |
| Xanthan gum | 0.3 | 0.2 | 0.4 | 0.4 | 0.2 |
| Cetearyl alcohol | 3 | | | | |
| Petrolatum | 1 | 0.1 | - | 1 | 5 |
| Mineral oil | - | 10 | 10 | - | - |
| Perfume | q.s. | q.s. | q.s. | q.s. | q.s. |
| Ethanol | 10 | 3 | 4 | 5 | 10 |
| Water | ad 100 | ad 100 | ad 100 | ad 100 | ad 100 |

### Example 33: Sprays

| | **1** | **2** | **3** | **4** | **5** |
|---|---|---|---|---|---|
| **Ingredient (INCI)** | **% by weight** | | | | |
| N-Acetylhydroxyproline | 5 | 3.0 | 2.0 | 1.5 | 2.5 |
| Hammamelis extract | 0.2 | --- | --- | 1 | 0.2 |
| Aloe vera extract | --- | --- | 1 | 1 | 5.0 |
| Tocopherol | 0.5 | 0.5 | 0.75 | 0.25 | 0.1 |
| Sea extract | 0.5 | --- | 0.75 | 3.0 | 0.25 |
| Panthenol | 0.5 | --- | 0.75 | 0.25 | 0.1 |
| Carbomer | 0.05 | 0.35 | --- | --- | --- |
| Perfume | q.s. | q.s. | q.s. | q.s. | q.s. |
| Sorbitol | 10 | --- | -- | 5 | --- |
| Butylene glycol | --- | --- | --- | 5 | 10 |
| Propylene glycol | --- | --- | 10 | 5 | --- |
| Glycerin | --- | 7.5 | --- | --- | --- |
| Water | ad 100 | ad 100 | ad 100 | ad 100 | ad 100 |

### Example 34: Effect of N-Acetylhydroxyproline on Sensory and Care Properties of Cosmetic O/W Creams

Two O/W (oil-in-water) creams of the following compositions were tested by eight panelists and compared with respect to various sensory and care properties.

| | **Invention** | **Comparison** |
|---|---|---|
| **Ingredient (INCI)** | **% by weight** | |
| Paraffinum Liquidum | 3 | 3 |
| Glyceryl Stearate | 1.2 | 1.2 |
| Stearic Acid | 2.5 | 2.5 |
| Water | 75.13 | 72.053 |
| Cera Microcristallina + Paraffinum Liquidum | 2 | 2 |
| Tocopheryl Acetate | 0.5 | 0.5 |
| Myristyl Alcohol | 3.5 | 3.5 |
| Phenoxyethanol + Methylparaben + Ethylparaben + Butylparaben + Isobutylparaben + Propylparaben | 0.35 | 0.35 |
| Dimethicone | 0.75 | 0.75 |
| Hydrogenated Coco-Glycerides | 1 | 1 |
| Simmondsia Chinensis Oil | 0.5 | 0.5 |
| Myristyl Myristate | 1.5 | 1.5 |
| Lanolin Alcohol (Eucerit®) | 0.25 | 0.25 |
| Glycerin | | 3.477 |
| Sodium Hydroxide | 2 | 0.6 |
| Butylene Glycol | | 3 |
| Alcohol Denat. | 3 | 3 |
| Carbomer | 0.3 | 0.3 |
| Polyglyceryl-2 Caprate | 0.35 | 0.35 |
| Parfum | 0.17 | 0.17 |
| N-Acetylhydroxyproline | 2 | |
| | 100 | 100 |

Seven of the eight panelists found the composition according to the present invention to be suitable for the care of chapped and brittle skin whereas none of the panelists considered the comparative composition to be suitable for this purpose. Further, six panelists found the composition according to the present invention to leave a less sticky feeling on the skin than the comparative composition and only one of the panelists rated the comparative composition to be better in this regard. Five of the panelists found the composition according to the present invention to reduce itching of the skin, compared to only one panelist in the case of the comparative composition.

### Example 35: Effect of N-Acetylhydroxyproline on Sensory and Use Properties of Cosmetic W/S Emulsions

Two W/S (water-in-silicone) emulsions of the following compositions were tested by nine panelists and compared with respect to various sensory and use properties.

| | **Comparison** | **Invention** |
|---|---|---|
| **Ingredient (INCI)** | **% by weight** | |
| Ethylparaben | 0.2 | 0.2 |
| Methylparaben | 0.2 | 0.2 |
| Water | 29.725 | 36.375 |
| Tocopheryl Acetate | 0.6 | 0.6 |
| Trisodium EDTA | 1 | 1 |
| BHT | 0.05 | 0.05 |
| Phenoxyethanol | 0.4 | 0.4 |
| Cyclomethicone | 8 | 8 |
| Butyl Methoxydibenzoylmethane | 4.5 | 4.5 |
| Phenylbenzimidazole Sulfonic Acid | 2.5 | 2.5 |
| VP/Hexadecene Copolymer | 0.5 | 0.5 |
| Glycerin | 6 | |
| Sodium Hydroxide | 1 | 2.4 |
| Alcohol Denat. | 2 | 2 |
| Cetyl PEG/PPG-10/1 Dimethicone | 0.5 | 0.5 |
| Ethylhexyl Salicylate | 2.25 | 2.25 |
| Butyrospermum Parkii | 3 | 3 |
| Titanium Dioxide + Trimethoxycaprylylsilane | 1.5 | 1.5 |
| Sodium Hyaluronate | 0.05 | |
| Lauroyl Lysine | 1 | 1 |
| Octocrylene | 5 | 5 |
| Homosalate | 2.25 | 2.25 |
| Bis-Ethylhexyloxyphenol Methoxyphenyl Triazine | 2.25 | 2.25 |
| Butylene Glycol Dicaprylate/Dicaprate | 8 | 8 |
| Cyclomethicone | 8 | 8 |
| Glycyrrhiza Inflata Root Extract | 0.025 | 0.025 |
| Methylpropanediol | 4 | |
| Polymethylsilsesquioxane | 2 | 2 |
| N-Acetylhydroxyproline | | 2 |
| Polyamide-5 | 3.5 | 3.5 |
| | 100 | 100 |

Six of the nine panelists found the composition according to the present invention to exhibit a better spreadabilty on the skin than the comparative composition whereas only two of the panelists considered the comparative composition to be better in this regard. Further, five panelists rated the composition according to the present invention to leave a less sticky feeling on the skin and to make the skin less shiny than the comparative composition whereas none of the panelists rated the comparative composition to be better in this regard. Five of the panelists found the composition according to the present invention to be better absorbed by the skin than the comparative composition, compared to only two panelists who considered the comparative composition to be better in this respect.

### Example 36: Effect of N-Acetylhydroxyproline on SPF Values of UV-Filter Containing Cosmetic Compositions

Two UV-Filter containing cosmetic compostions of the following compostions were tested with respect to SPF values according to the COLIPA method ("METHOD FOR THE IN VITRO DETERMINATION OF UVA PROTECTION PROVIDED BY SUNSCREEN PRODUCTS", Guideline 2007a, prepared by the COLIPA In vitro Photoprotection Method Task Force, the entire disclosure whereof is incorporated by reference herein):

| | **Comparison** | **Invention** |
|---|---|---|
| **Ingredients (INCI)** | **% by weight** | |
| Methylparaben | 0.3 | 0.3 |
| Water | 52.215 | 43.715 |
| Trisodium EDTA | 1 | 1 |
| Ethylhexyl Methoxycinnamate + BHT | 0.5 | 0.5 |
| Phenoxyethanol | 0.6 | 0.6 |
| Dimethicone | 2 | 2 |
| Hydrogenated Coco-Glycerides | 3 | 3 |
| C12-15 Alkyl Benzoate | 6 | 6 |
| Butyl Methoxydibenzoylmethane | 4.5 | 4.5 |

| | | |
|---|---|---|
| Glycerin | 4.3 | 4.3 |
| Sodium Hydroxide | 0.185 | 3.685 |
| Alcohol Denat. | 4 | 4 |
| Xanthan Gum | 0.4 | 0.4 |
| Titanium Dioxide + Trimethoxycaprylylsilane | 3 | 3 |
| Stearyl Alcohol | 3 | 3 |
| Glyceryl Stearate Citrate | 2 | 2 |
| Ethylhexylglycerin | 0.5 | 0.5 |
| Octocrylene | 8 | 8 |
| Bis-Ethylhexyloxyphenol Methoxyphenyl Triazine | 1.5 | 1.5 |
| Diethylhexyl Butamido Triazone | 1 | 1 |
| Butylene Glycol Dicaprylate/Dicaprate | 2 | 2 |
| N-Acetylhydroxyproline | 0 | 5 |
| | | |
| | 100 | 100 |

The test results were as follows:

### In vitro SPF:

Comparison: 78.1
Invention: 81.3 (increase relative to comparative composition: 4.1 %)

### UVA SPF:

Comparison: 13.3
Invention: 14.4 (increase relative to comparative composition: 8.3 %)

COLIPA Ratio (UVA protection vs. UVB protection; ratio should be as low as possible and should in any event be lower than 3):
Comparison: 2.3
Invention: 2.1 (improvement by 9.5 % relative to comparative composition).

### Example 37: Lightening Effect of N-Acetylhydroxyproline on Undesired Skin Pigmentation

Undesired and uneven skin pigmentation such as, e.g., age spots (senile lentigo) are a major problem associated with the aging of skin and skin appendages. N-acetylhydroxyproline has an effect with respect to the lightening of age spots. For example, after seven days the age spot treated with N-acetylhydroxyproline showed an increase in brightness.

### Example 38: Determination of modification sites of vimentin

The following methods and procedures where used in the determination of the modicification sites of vimentin described above:

### Cell culture

Human skin biopsies were isolated from healthy donors. Primary human dermal cells were enzymatically prepared using a standardised dispase (Boehringer Mannheim, Mannheim, Germany) digestion technique. The dermal fraction was cultured at 37°C and 7% CO₂ (in air) in six-well plates containing Dulbecco's modified Eagle's medium (Life Technologies, Eggenstein, Germany) supplemented with 10% foetal calf serum (Life Technologies) and penicillin/streptomycin (50 µg/ml, Life Technologies). After 5-6 weeks of incubation, confluent fibroblasts were seeded into the appropriate flasks. The keratinocytes of the epidermal fraction were cultured in KGM-2 (Cambrex, Apen, Germany).

Additional vimentin⁻ and vimentin⁺ fibroblasts, derived from murine embryos, were employed. Vimentin⁻ and vimentin⁺ fibroblasts were grown with additional 10 µg/ml gentamicin (GIBCO, Auckland, New Zealand). Fibroblasts expressing vimentin-GFP³³ were employed as well. The vimentin-GFP cells were grown with additional 300 µg/ml geneticin (GIBCO).

### Immunoblotting analysis

Proteins from cell extracts (20 µg per lane) were separated by gel electrophoresis under denaturing conditions on 10% SDS-PAGE gels. Immunoblotting was performed following standard procedures using chemiluminescence methods. Protein bands, blotted on nitrocellulose or PVDF sheets, were visualised with the Lumilight Plus Western Blot Detection Kit (Roche, Mannheim, Germany). Imaging was performed using LUMI-Imager (Boehringer, Mannheim, Germany). Signals were quantified using LumiAnalyst (Boehringer Mannheim, Germany). The CML⁵⁰ antibody used was clone 6D12 (TransGenic Inc., Kumamoto, Japan, 1:200 dilution). Antibodies detecting vimentin (sc-7558 dilution 1:200, sc-32322 dilution 1:100,000) and actin (sc-1615 dilution 1:500) were purchased from Santa Cruz Biotechnology (California, USA). Antibody detecting GAPDH (clone 5G4 dilution 1:500) was purchased from HyTest (Turku, Finland). Secondary antibodies labelled with peroxidase were purchased from Sigma Aldrich (A6782 dilution 1:1,000, A8919 dilution 1:1,000, St. Louis, USA).

### Two-dimensional electrophoresis

20-100 µg protein was used for the isoelectric focusing. This amount of protein was precipitated from lysates using ice cold trichloroacetic acid. The pellet was resuspended in rehydration buffer (8 M urea, 2% CHAPS, 0.002% bromophenol blue, 18 mM DTT). The rehydration buffer was supplemented with 0.2% Bio-Lyte (Bio-Rad Laboratories, USA). The pH 3-10 IPG-strips were covered with this solution and with silicon oil. The rehydration was performed using a PROTEAN®IEF-Cell (50 V, 12 h) followed by the IEF. The IPG-strip was equilibrated for 15 minutes in equilibration buffer (50 mM Tris-HCl, 6 M urea, 30% glycerol, 2% SDS, 0.002% bromophenol blue) supplemented with 65 mM dithiothreitol followed by a 15 minute incubation in equilibration buffer with 135 mM iodoacetamide. The separation was performed in 10% polyacrylamide gels using standard electrophoresis buffer (250 mM Tris-HCL, 1.9 M glycine, 1% SDS). Preparative gels for mass spectrometric analysis were stained with 0.2% Coomassie blue R-250.

### Cell-Monolayer immunohistochemistry

For immunostaining, cells were fixed in 3% paraformaldehyde for 30 minutes at room temperature, washed with PBS and permeabilized with 0.5% Triton X-100 for 5 minutes, after which the cells were blocked with 3% BSA for 30 minutes. Cells were then incubated with primary antibody in 1% BSA for 1 hour. Antibodies detecting vimentin (sc-7558 dilution 1:100, sc-32322 dilution 1:200), actin (sc-1615 dilution 1:100) and CFTR (sc-8910 dilution 1:100) were purchased from Santa Cruz Biotechnology (California, USA). The antibody detecting CML⁵⁰ (6D12, dilution 1:50) was purchased from TransGenic Inc. (Kumamoto, Japan). After this incubation the cells were washed with 0.05% NP-40 and PBS. Cells were then incubated for 1 hour with 1% BSA solution containing fluorescence-labelled secondary antibodies (AlexaFluor 488 chicken-anti-mouse IgG, AlexaFluor 594 donkey-anti-goat IgG, AlexaFluor 546 donkey-anti-goat IgG, Molecular Probes, Eugene, Oregon) at 1:1,000 to visualise the target protein and DAPI (Molecular Probes, Eugene, Oregon). After extensive washing with PBS fluorescence images were recorded on a fluorescence microscope (Olympus, Hamburg, Germany) with an attached closed-circuit display camera. Confocal fluorescence images were recorded on LSM 510 Meta (Carl Zeiss, Jena, Germany).

### Immunoprecipitation and cytoskeletal fractionation studies

50 µl of a vimentin agarose conjugate (sc-7558AC, Santa Cruz Biotechnology) was added to 500 µl of 2x immunoprecipitation buffer (300 mM NaCl, 20 mM Tris-HCl, 2 mM EDTA, 2 mM EGTA, 0.4 mM PMSF, 1% NP-40, 2% Triton X-100) and 400 µl ddH₂O. 100 µl lysate was added containing 100-500 µg protein. This solution was incubated at 4 degrees for 1 hour followed by centrifugation. The pellet was washed several times with 1x immunoprecipitation buffer, resuspended and boiled for 5 minutes in 30 µl 2x Laemmli buffer (4% SDS, 20% glycerol, 10% 2-mercapthoethanol, 0.004% bromophenol blue, 0.125 M Tris HCl, pH 6.8). Every centrifugation was performed at 4 degrees and 13,000 rpm.

The fractionation was done with the ProteoExtract^{(R)}Subcellular Proteome Extraction Kit (Merck, Darmstadt, Germany).

### nanoLC-ESI-MS/MS

Protein identification using nanoLC-ESI-MS/MS was performed by Proteome Factory (Proteome Factory AG, Berlin, Germany). The MS system consisted of an Agilent 1100 nanoLC system (Agilent, Boeblingen, Germany), PicoTip emitter (New Objective, Woburn, USA) and an Esquire 3000 plus ion trap MS (Bruker, Bremen, Germany). Protein spots were in-gel digested by trypsin (Promega, Mannheim, Germany) or thermolysine (Fluka, Seelze, Germany) and applied to nanoLC-ESI-MS/MS. After trapping and desalting the peptides on enrichment column (Zorbax SB C18, 0.3 x 5 mm, Agilent) using 3% acetonitril/0.1% formic acid solution for five minutes peptides were separated on Zorbax 300 SB C18, 75 µm x 150 mm column (Agilent) using an acetonitril/0.1% formic acid gradient from 5% to 40% acetonitril within 40 minutes. MS spectra were automatically taken by Esquire 3000 plus according to manufacturer's instrument settings for nanoLS-ESI-MSMS analyses. Proteins were identified using MS/MS ion search of Mascot search engine (Matrix Science, London, England) and nr protein database (National Center for Biotechnology Information, Bethesda, USA).

### FACS analysis

Fibroblasts were incubated for 7 days with 200 µM glyoxal^{18,20}. The trypsinized cells were centrifuged at 13,000 rpm and washed twice with PBS. The resulting pellet was resuspended in 3% PFA and incubated for 30 minutes at room temperature. After 2 additional washing steps the cells were permeabilized with 0.5% Triton-X100 (Sigma, St. Louis, USA) in PBS, washed again and blocked with 3% BSA (Roth, Karlsruhe, Germany) in PBS for 30 minutes at room temperature. FITC-labelled antibody detecting CML⁵⁰ (Transgenic Inc., Kumamoto, Japan, 1:100 dilution) was added in 1% BSA and incubated for 1 hour at room temperature. After 3 washing steps with PBS the fluorescence was measured using FACSCanto (BD Biosciences, San Jose, USA) and analysed by the software BD FACSDiva 4.0.

### Contraction capacity studies

100 mg Type I collagen (Sigma, St. Louis, USA) was dissolved at 4°C in 33.3 ml of 0.1% sterile acetic acid. 0.9 ml of 10x Hank's Buffer Salt Solution (Biochrom, Berlin, Germany) was added to 4.5 ml of this solution of collagen. 1.9 ml of 0.1% acetic acid was supplemented. NaOH was added dropwise to neutralise the solution. 1.5 x 10⁵ fibroblasts were resuspended in 0.1 ml FCS and added to the solution for each ml of the gel. Casy Model TT (Schärfe System, Reutlingen, Germany) was used to count the fibroblasts and to control their viability. 2 ml of the final collagen solution containing the fibroblasts were applied to six-well plates and incubated at 37°C and 7% CO₂ (in air) for 1 hour. The gels were covered with 2 ml DMEM and incubated for another 10 hours at 37°C and 7% CO₂ (in air). To examine the contraction capacity, gels were detached from the side of the wells and further incubated. Diameters were measured at 0 hours and 24 hours. The methodical approach of collagen lattices is based on Bell et al.³⁰ and was described by Delvoye et al.²⁹ for measuring contraction in fibroblasts.

### Paraffin section immunohistochemistry

Briefly, skin biopsies from healthy human donors were incubated for 2 hours in 4% paraformaldehyde followed by incubation in water for 3 hours. The biopsies were dehydrated with ethanol and isopropanol followed by the histoprocessing at 80°C and the embedding in paraffin. 5 µm sections were prepared and the paraffin was released using xylol. After stepwise rehydration sections were blocked with 1% BSA, washed with PBS and incubated for 1 hour with the primary antibody followed by additional washes with PBS. Afterwards the incubation for 1 hour with the secondary antibody was performed. Past extensive washing the imaging was started on a fluorescence microscope (Olympus, Hamburg, Germany).

### Statistical analysis

Statistical data were analysed using Statistica 7.1 (StatSoft, Tulsa, USA). Normality was tested performing the Lilliefors-test. Statistical significance was determined using the two-tailed unpaired t-test.

Abbreviations: AGEs, advanced glycation endproducts; CFTR, cystic fibrosis transmembrane conductance regulator; CML, N^{ε}-(carboxymethyl)lysine; DAPI, 4',6-diamidine-2'-phenylindole; ESI, electrospray ionisation; FCS, foetal calf serum; FITC, fluorescein isothiocyanate; GAPDH, glyceraldehyde-3-phosphate dehydrogenase; GFAP, glial fibrillary acidic protein; GFP, green fluorescent protein; IPG, immobilized pH gradient; LC, liquid chromatography; MS/MS, tandem mass spectrometry; PBS, phosphate buffered saline; PFA, paraformaldehyde; SDS-PAGE, sodium dodecyl sulfate polyacrylamide gel electrophoresis;

### Legends to Figures 1-8:

### Figure 1 Identification of vimentin as a major target for CML modification in primary human fibroblasts

Primary human fibroblasts and keratinocytes of 3 different donors were isolated, cultured and lysed as described in the Methods section. Cell lysates were separated by SDS-PAGE and analysed using an anti-CML antibody^{18,50} (a). The lanes represent the analysis of three donors using keratinocyte (K) or fibroblast (F) lysates. The right lane shows the silver staining pattern of a fibroblast lysate of donor C. Fibroblast lysates were analysed by 2DE followed by immunoblotting (b). The position of the CML (upper panel) signal with the position of the signal for vimentin (lower panel) was compared. The colocalisation of vimentin and CML was examined using immunofluorescence staining as described in the Methods section (c). Lysates of fibroblasts were fractionated as described above. The cytoskeletal fraction (CF) was separated by SDS-PAGE and either silver stained (stain) or immunoblotted with an α-CML-antibody (α-CML). Additionally an immunoprecipitation of vimentin was performed (IP). The bands of the CF were identified as actin (A) and vimentin (V) using the corresponding antibodies.

### Figure 2 Slow turnover of vimentin is not the reason for strong CML modification

Primary human fibroblasts were cultured as described above, with the exception that 40 µg/ml cycloheximide were added at time point 0. Cells were harvested at 0, 24, 48 h and analysed by SDS-PAGE and immunoblotting with α-vimentin (V) and α-actin (A) antibodies (a) or α-vimentin (V) and α-GAPDH (G) antibodies (b) Comparative quantitative analysis was performed by analysing signal intensities of 7-10 immunoblots. The initial signal intensity was set as 100% for each of the experiments. The data are Mean ± S.D. with (*) p< 0.05, (**)p<0.01.

### Figure 3 Exposed linker regions of vimentin are preferential targets for CML modification

Vimentin was isolated from lysates of fibroblasts by fractionation and analysed by nanoLC-ESI-MS/MS. The vimentin sequence of the NCBI-database (NP_003371.2) was used. In panel (a) the lysines found CM-modified using nanoLC-ESI-MS/MS are shown in red (K). The unmodified lysines are shown in black (K). The linker regions of vimentin²⁴ are highlighted in grey. (b) The modified lysines of the hinge regions are shown in the structural context of the protein vimentin. The structure is based on Strelkov et al.²⁴. The rod domain consists of the coiled-coil domains 1A, 1B, 2A, 2B which are separated by the linker L1, L12 and L2. The linker L2 does not contain any lysine that could potentially be modified.

### Figure 4 Murine vimentin-knockout fibroblasts exhibit reduced levels of CML modifications

Vimentin (+/+) and vimentin (-/-) fibroblasts were cultured, harvested and analysed by FACS or dot-blot analysis for CML-content. For FACS analysis a fluorescence labelled antibody detecting CML was used (a). Quantitative analysis of the FACS data is shown in (b, left columns). Right columns in panel (b) represent the quantitative analysis of dot blots detecting the CML level of these two populations. The signal intensity for vimentin (+/+) was set as 100%. Each column represents the mean of 5 independent measurements.

### Figure 5 CML formation contributes to the loss of contractile capacity of human fibroblasts

Human fibroblasts were incubated for 7 days with 200 µM glyoxal^{18,20} and tested for CML formation using FACS analysis as described above (a). Quantitative analysis of (a) is shown in panel (b, right columns) together with quantitative analysis of dot-blots as described in the legend to Fig.4 and the Methods section (b, left columns). All columns are the mean of 5 independent experiments, with the signal from untreated cells set as 100%. Additionally, the two cell populations were lysed, vimentin was immunoprecipitated and analysed for CML-modification by SDS-page and immunoblotting. The two bands on the right display precipitated vimentin blotted for CML with (w) and without (w/o) glyoxal treatment (b, right part). Glyoxal treated fibroblasts and control fibroblasts were seeded into 3D collagen lattices. The contraction of these lattices was measured after 48h. The results of 10 analyses are displayed in panel (c) in a Box-Whisker-Plot. The contractile capacity of the glyoxal treated fibroblasts (n=10) was significantly reduced; p=0.00056. In (d) two collagen lattices are shown exemplarily. The left lattice contains control fibroblasts (w/o Glyoxal). The right lattice contains glyoxal-treated fibroblasts (Glyoxal).

### Figure 6 CML induces redistribution of the filament vimentin to a perinuclear aggregate

Monolayer of primary human fibroblasts were stained and imaged for vimentin after 2 days of glyoxal treatment (a) as described above. Cells with glyoxal treatment (Glyoxal) and without treatment (w/o Glyoxal) were analysed for vimentin (green) and actin (red) distribution. Nuclei were DAPI-stained (blue). Redistribution of GFP-vimentin was followed in a time dependent manner. Confocal images of panel (b) illustrate the distribution of the vimentin filament in a wide-spread network (top) without glyoxal treatment (w/o Glyoxal) or in a dense aggregate (bottom) after 2 days of glyoxal treatment (Glyoxal). Cells expressing GFP-vimentin (see Methods) were treated at time point 0 with glyoxal and analysed at the indicated time points by confocal fluorescence microscopy (c). The redistribution of the vimentin filament from a wide-spread network (0 h) to a dense aggregate (16 h) was observed. Arrows indicate the forming aggregate.

### Figure 7 Aggregates contain CML and the aggresome marker CFTR

The vimentin aggregates identified in Fig.6 were analysed for CML-content. Samples were prepared as described above. Panel (a) shows a monolayer of glyoxal treated fibroblasts stained for CML (red) and vimentin (green) to test for colocalisation in the aggregate. A DAPI staining was performed (blue). In panel (b) the test for colocalisation with the aggresome marker cystic fibrosis transmembrane conductance regulator (CFTR)³¹ was performed. Control fibroblasts (w/o Glyoxal) and glyoxal treated fibroblasts (Glyoxal) were stained for the aggresome marker CFTR (red) and vimentin (green) to investigate, whether the aggregate consists of an aggresome³¹. In parallel a DAPI staining was performed (blue).

### Figure 8 Vimentin forms aggresomes in vivo

A facial skin biopsy of a 76 year old male donor was analysed. The biopsy was embedded in paraffin. Sections of 5 µm were prepared and stained for vimentin (green) to examine whether aggregated vimentin can be *detected in vivo.* Cell nuclei are stained with DAPI (blue). The identified aggresomes are indicated by arrows. The upper right panel shows the negative control stained only with secondary antibody.

### Example 39: Determination of effectiveness in inhibiting the formation of AGEs

The following experiments were carried out to test the effectiveness of various compounds in inhibiting the formation of AGEs.

The following abbreviations are used:
- BSA: Bovine Serum Albumin
- CML: Carboxymethyl lysine
- DHA: Dihydroxyacetone

### A: Inhibition of BSA-CML-Formation with Increasing NAHP-Concentration:

### Protocol:

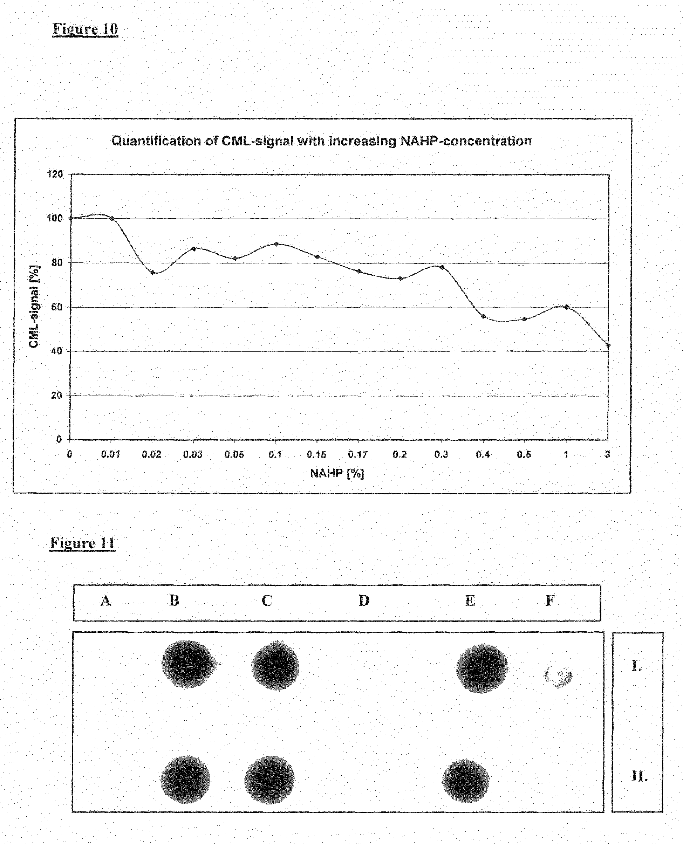

BSA samples of were incubated with glucose for 7 days at 50°C in the presence of various amounts of NAHP and thereafter analysed for their CML-content using a dot-blot approach. An antibody which is specific for the CML modifications of immobilised proteins (in this case BSA) on a nitrocellulose membrane was used to make the CML modifications visible. Figure 9 shows the dot-blot results. The stronger the intensity of each dot in the above series, the higher is the concentration of CML-modifications on the BSA. The control consists of BSA without glycation agent (glucose). Each single dot was quantified for its intensity leading to the results which are shown diagrammatically in Figure 10. As can be seen from the these results, NAHP displays a protective effect on BSA, preventing the CML glycation of this protein. Increasing concentrations of NAHP exhibit an increasing protective effect on BSA.

The following table shows the concentration of NAHP in each sample used for the dot-blot approach. The concentration of BSA was 1.035 % (10.35 mg/ml) and the concentration of glucose in each sample was 1 M.

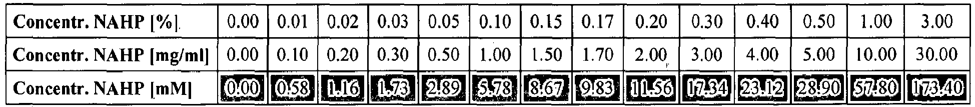

### B: Comparison of NAHP with Structurally Similar Compounds Retarding the Inhibitory Effect on AGE-Formation with DHA:

### Protocol:

Samples (A-F) were incubated for 1 day at 50°C and analysed for CML-content using the dot-blot approach set forth above in A. The dot-blot results are shown in Figure 11.

The samples had the following composition:
**A:** only BSA (1%)
**B:** BSA (1%) with DHA (1%)
**C:** BSA (1%) with Hydroxyproline and DHA (1%)
**D:** BSA (1%) with N-Acetylhydroxyproline (NAHP) and DHA (1%)
**E:** BSA (1%) with Proline and DHA (1%)
**F:** BSA (1%) with Acetylproline and DHA (1%)

Each sample (A-F) was tested twice (I+II) with the difference that I. was conducted with 0.17 % of each inhibitor (Proline, Hydroxyproline, Acetylproline or NAHP) and II. was conducted with 9.82 mM of each inhibitor.

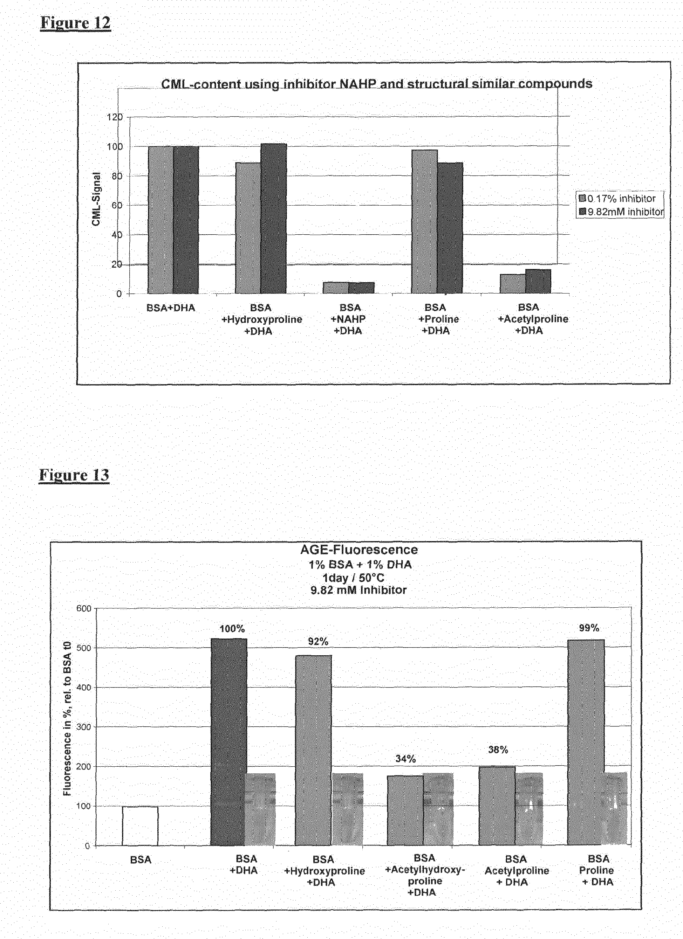

The intensity of each dot was quantified. The results are represented in Figure 12. As can be seen, NAHP exhibits the strongest inhibitory effect on the CML-glycation of BSA in both series (I+II). Acetylproline is also a potent inhibitor of the CML-glycation, but to a lesser extent than NAHP.

Samples A-F (II) were further tested regarding the inhibition of the formation of other AGEs (different from CML). To this end, the AGE-fluorescence at λₑₓ=370 nm and λₑₘ= 440 nm was measured. The wavelengths used are those proposed by Bellmunt et al., Lung 173 (1995), 177-85 and used by many laboratories. The results are shown in Figure 13. The fluorescence data prove that not only the formation CML but also the formation of fluorescent AGEs is inhibited by NAHP. The digital picture next to each column shows that there is also a difference in the color of the different solutions, which difference is partly due to the brownish color of AGEs which is absent in the solutions containing NAHP.

### C: Comparison of NAHP with Structurally Similar Compounds Regarding the Inhibitory Effect on AGE-Formation with Glucose:

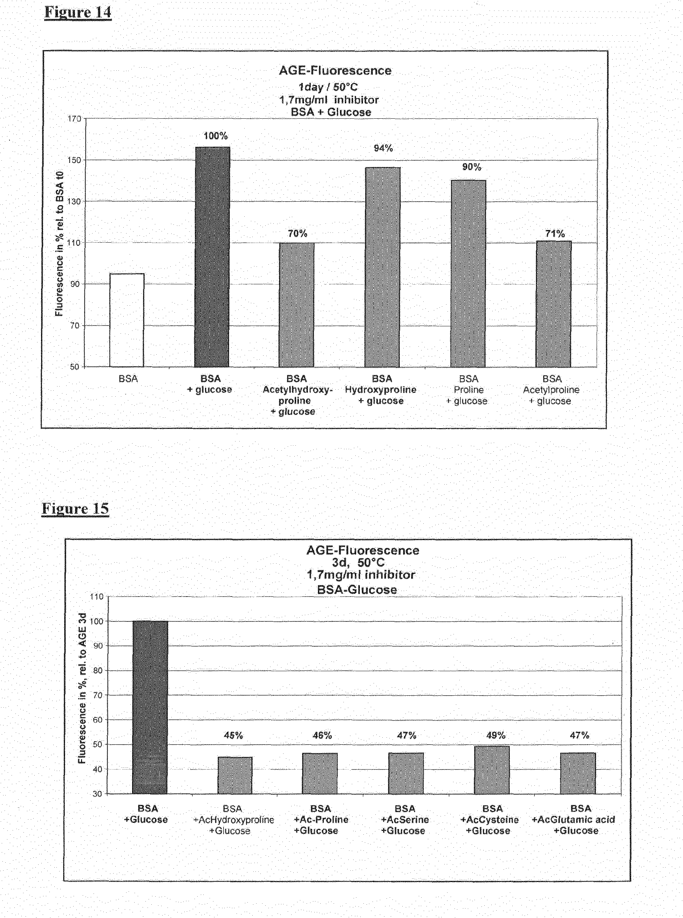

The results set forth above under B were confirmed using glucose as glycation agent. Figure 14 shows the AGE-fluorescence of each tested sample. The composition of each sample was the same as in B which the exception that DHA was replaced by glucose.

### D: Comparison of Inhibition of AGE Formation by NAHP and Other Acetylated Amino Acids

N-Acetylhydroxyproline was compared to other acetylated amino acids with respect to the capability of inhibiting the AGE-formation in the BSA-glucose assay. Figure 15 shows the results of this experiment. The incubation time was 3 days at 50°C. The value for BSA+Glucose without inhibitor was taken as 100%.

### E: Inhibition of AGE-Formation by Other Acetylated Compounds

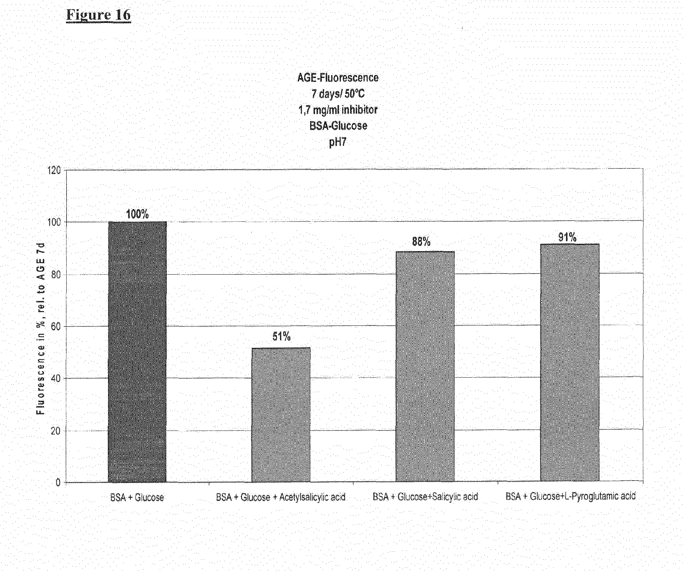

Using glucose as glycation agent other actylated compounds which are structurally related to NAHP were tested for their inhibitory potential with respect to AGE-formation in the BSA-glucose assay. The samples were incubated for 7 days at 50°C, the AGE detection was performed following the procedure set forth above in A. Figure 16 shows the obtained results.

The BSA-Glucose control without inhibitor was taken as 100%. The obtained results indicate that acetyl groups are likely involved in the inhibitory effect of AGE-formation. All tested compounds resulted in reduced AGE-fluorescence, with acetylsalicylic acid having the strongest effect.

### F: AGE-Inhibition with NAHP Enhances the pH-Modulated Inhibitory Effect

As acetylated compounds result in a decrease in the pH, the effect of NAHP was compared at two different pH values (pH 5 and pH 7). As described in Section A, the BSA-Glucose Assay was used. To stabilize the pH the adjustment was performed after the addition of the inhibitor.

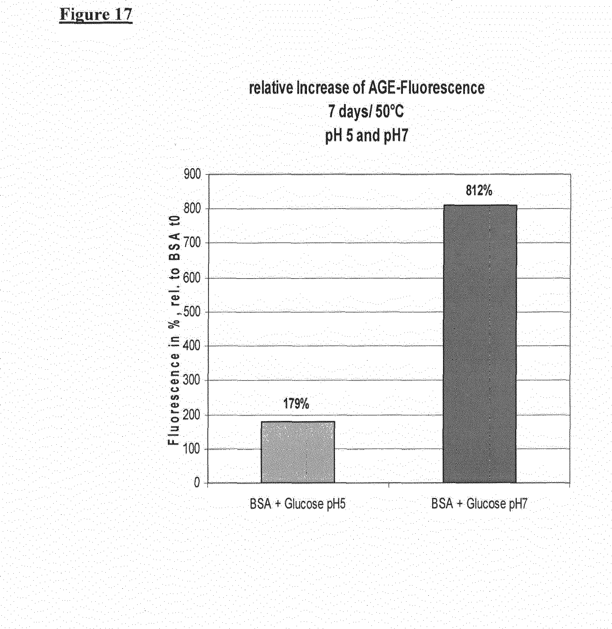

As illustrated in Figure 17, the pH has a distinct effect on AGE-formation. After incubation of BSA with glucose for 7 days at 50°C a significant increase in AGE fluorescence is detectable. An acidic environment (pH 5) results in a much less intensive AGE-formation in the BSA-Glucose Assay than a neutral environment (pH 7).

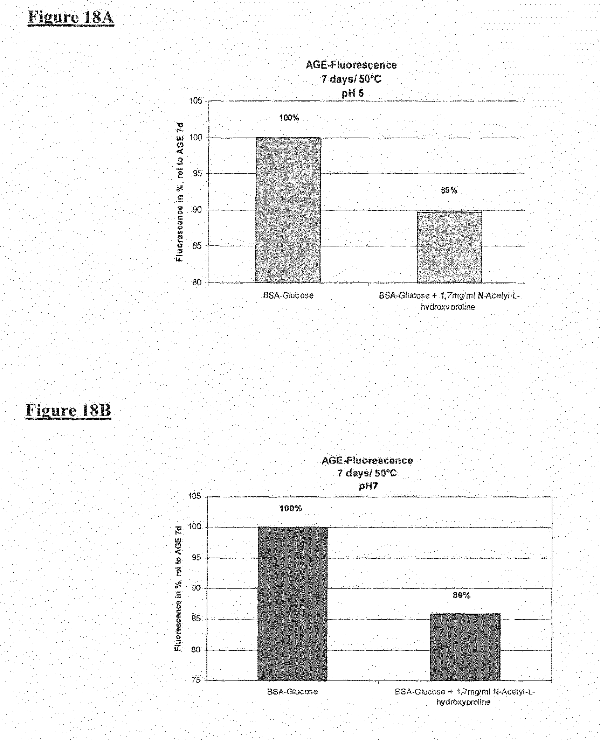

Incubation with N-acetylhydroxyproline reduced AGE-formation at both tested pH values (see Figures 18A and 18B). These results demonstrate that AGE formation can be inhibited by N-acetylhydroxyproline both at pH 5 and pH 7.

It is noted that the foregoing examples have been provided merely for the purpose of explanation and are in no way to be construed as limiting of the present invention. While the present invention has been described with reference to an exemplary embodiment, it is understood that the words that have been used are words of description and illustration, rather than words of limitation. Changes may be made, within the purview of the appended claims, as presently stated and as amended, without departing from the scope and spirit of the present invention in its aspects. Although the invention has been described herein with reference to particular means, materials and embodiments, the invention is not intended to be limited to the particulars disclosed herein. Instead, the invention extends to all functionally equivalent structures, methods and uses, such as are within the scope of the appended claims.

The entire disclosures of the following documents are expressly incorporated by reference herein:
U.S. Patent Nos. 3,997,559; 3,932,638; 6,159,485; 6,497,889; 6,524,593; 6,692,754; and 7,138,386;
published U.S. Patent Application Nos. 2003/0185864; 2006/0034781; and 2006/0035957;
EP 1 159 952 A1 and EP 0 415 598 A1,
WO 97/38690,
FR 2 619 711 and FR 2 800 990,
JP 2004-315384 and JP 2005-145929.

1. Jeanmaire, C., Danoux, L. & Pauly, G. Glycation during human dermal intrinsic and actinic ageing: an in vivo and in vitro model study. Br J Dermatol 145, 10-8 (2001).
2. Bucciarelli, L. G. et al. RAGE is a multiligand receptor of the immunoglobulin superfamily: implications for homeostasis and chronic disease. Cell Mol Life Sci 59, 1117-28 (2002).
3. Vlassara, H. Advanced glycation in health and disease: role of the modern environment. Ann N YAcad Sci 1043, 452-60 (2005).
4. Cloos, P. A. & Christgau, S. Non-enzymatic covalent modifications of proteins: mechanisms, physiological consequences and clinical applications. Matrix Biol 21, 39-52 (2002).
5. Schmidt, A. M., Yan, S. D. & Stern, D. M. The dark side of glucose. Nat Med 1, 1002-4 (1995).
6. Dunn, J. A., McCance, D. R., Thorpe, S. R., Lyons, T. J. & Baynes, J. W. Age-dependent accumulation of N epsilon-(carboxymethyl)lysine and N epsilon-(carboxymethyl)hydroxylysine in human skin collagen. Biochemistry 30, 1205-10 (1991).
7. Dunn, J. A., Patrick, J. S., Thorpe, S. R. & Baynes, J. W. Oxidation of glycated proteins: age-dependent accumulation of N epsilon-(carboxymethyl)lysine in lens proteins. Biochemistry 28, 9464-8 (1989).
8. Baynes, J. W. The role of AGEs in aging: causation or correlation. Exp Gerontol 36, 1527-37 (2001).
9. Bucala, R. & Cerami, A. Advanced glycosylation: chemistry, biology, and implications for diabetes and aging. Adv Pharmacol 23, 1-34 (1992).
10. van Heijst, J. W. et al. Argpyrimidine-modified Heat Shock Protein 27 in human non-small cell lung cancer: A possible mechanism for evasion of apoptosis. Cancer Lett 241, 309-19 (2006).
11. Ulrich, P. & Cerami, A. Protein glycation, diabetes, and aging. Recent Prog Horm Res 56, 1-21 (2001).
12. Dyer, D. G. et al. Accumulation of Maillard reaction products in skin collagen in diabetes and aging. J Clin Invest 91, 2463-9 (1993).
13. Degenhardt, T. P., Thorpe, S. R. & Baynes, J. W. Chemical modification of proteins by methylglyoxal. Cell Mol Biol (Noisy-le-grand) 44, 1139-45 (1998).
14. Shinohara, M. et al. Overexpression of glyoxalase-I in bovine endothelial cells inhibits intracellular advanced glycation endproduct formation and prevents hyperglycemia-induced increases in macromolecular endocytosis. J Clin Invest 101, 1142-7 (1998).
15. Brownlee, M. Biochemistry and molecular cell biology of diabetic complications. Nature 414, 813-20 (2001).
16. Suzuki, K., Koh, Y. H., Mizuno, H., Hamaoka, R. & Taniguchi, N. Overexpression of aldehyde reductase protects PC 12 cells from the cytotoxicity of methylglyoxal or 3-deoxyglucosone. J Biochem (Tokyo) 123, 353-7 (1998).
17. Thornalley, P. J. The glyoxalase system: new developments towards functional characterization of a metabolic pathway fundamental to biological life. Biochem J 269, 1-11 (1990).
18. Kasper, M. & Funk, R. H. Age-related changes in cells and tissues due to advanced glycation end products (AGEs). Arch Gerontol Geriatr 32, 233-243 (2001).
19. Stolzing, A., Widmer, R., Jung, T., Voss, P. & Grune, T. Degradation of glycated bovine serum albumin in microglial cells. Free Radic Biol Med 40, 1017-27 (2006).
20. Cervantes-Laurean, D., Roberts, M. J., Jacobson, E. L. & Jacobson, M. K. Nuclear proteasome activation and degradation of carboxymethylated histones in human keratinocytes following glyoxal treatment. Free Radic Biol Med 38, 786-95 (2005).
21. Fujii, T. et al. Ultrastructural changes and immunohistochemical localization of advanced glycation end products in the heart of streptozotocin-treated Mongolian gerbils. Med Electron Microsc 32, 43-49 (1999).
22. Reichenberg, E. et al. Proteomic analysis of protein components in periodontal ligament fibroblasts. J Periodontol 76, 1645-53 (2005).
23. Ahmed, N., Dobler, D., Dean, M. & Thornalley, P. J. Peptide mapping identifies hotspot site of modification in human serum albumin by methylglyoxal involved in ligand binding and esterase activity. J Biol Chem 280, 5724-32 (2005).
24. Strelkov, S. V. et al. Conserved segments 1A and 2B of the intermediate filament dimer: their atomic structures and role in filament assembly. Embo J 21, 1255-66 (2002).
25. Eckes, B. et al. Impaired mechanical stability, migration and contractile capacity in vimentin-deficient fibroblasts. J Cell Sci 111 ( Pt 13), 1897-907 (1998).
26. Smith, T. A., Strelkov, S. V., Burkhard, P., Aebi, U. & Parry, D. A. Sequence comparisons of intermediate filament chains: evidence of a unique functional/structural role for coiled-coil segment 1A and linker L1. J Struct Biol 137, 128-45 (2002).
27. Strelkov, S. V. & Burkhard, P. Analysis of alpha-helical coiled coils with the program TWISTER reveals a structural mechanism for stutter compensation. J Struct Biol 137, 54-64 (2002).
28. Wells-Knecht, K. J., Zyzak, D. V., Litchfield, J. E., Thorpe, S. R. & Baynes, J. W. Mechanism of autoxidative glycosylation: identification of glyoxal and arabinose as intermediates in the autoxidative modification of proteins by glucose. Biochemistry 34, 3702-9 (1995).
29. Delvoye, P., Wiliquet, P., Leveque, J. L., Nusgens, B. V. & Lapiere, C. M. Measurement of mechanical forces generated by skin fibroblasts embedded in a three-dimensional collagen gel. J Invest Dermatol 97, 898-902 (1991).
30. Bell, E., Ivarsson, B. & Merrill, C. Production of a tissue-like structure by contraction of collagen lattices by human fibroblasts of different proliferative potential in vitro. Proc Natl Acad Sci U S A 76, 1274-8 (1979).
31. Johnston, J. A., Ward, C. L. & Kopito, R. R. Aggresomes: a cellular response to misfolded proteins. J Cell Biol 143, 1883-98 (1998).
32. Kopito, R. R. Aggresomes, inclusion bodies and protein aggregation. Trends Cell Biol 10, 524-30 (2000).
33. Ho, C. L., Martys, J. L., Mikhailov, A., Gundersen, G. G. & Liem, R. K. Novel features of intermediate filament dynamics revealed by green fluorescent protein chimeras. J Cell Sci 111 ( Pt 13), 1767-78 (1998).
34. Grune, T., Jung, T., Merker, K. & Davies, K. J. Decreased proteolysis caused by protein aggregates, inclusion bodies, plaques, lipofuscin, ceroid, and 'aggresomes' during oxidative stress, aging, and disease. Int J Biochem Cell Biol 36, 2519-30 (2004).
35. Radfar, A. J. et al. Transplantation of virally transduced cells into the dermis of immunocompetent and immunodeficient (SCID) mice to determine gene expression profile and differential donor cell survival. Wound Repair Regen 8, 503-10 (2000).
36. Kim, S. S., Gwak, S. J., Choi, C. Y. & Kim, B. S. Skin regeneration using keratinocytes and dermal fibroblasts cultured on biodegradable microspherical polymer scaffolds. JBiomed Mater Res B Appl Biomater 75, 369-77 (2005).
37. Herrmann, H., Haner, M., Brettel, M., Ku, N. O. & Aebi, U. Characterization of distinct early assembly units of different intermediate filament proteins. J Mol Biol 286, 1403-20 (1999).
38. Munch, G. et al. Amino acid specificity of glycation and protein-AGE crosslinking reactivities determined with a dipeptide SPOT library. Nat Biotechnol 17, 1006-10 (1999).
39. Chang, L. & Goldman, R. D. Intermediate filaments mediate cytoskeletal crosstalk. Nat Rev Mol Cell Biol 5, 601-13 (2004).
40. Goedert, M. Parkinson's disease and other alpha-synucleinopathies. Clin Chem Lab Med 39, 308-12 (2001).
41. Johnson, A. B. & Bettica, A. On-grid immunogold labeling of glial intermediate filaments in epoxy-embedded tissue. Am J Anat 185, 335-41 (1989).
42. Zhou, J. et al. Mechanisms for the induction of HNE- MDA- and AGE-adducts, RAGE and VEGF in retinal pigment epithelial cells. Exp Eye Res 80, 567-80 (2005).
43. Brown, R. A., Talas, G., Porter, R. A., McGrouther, D. A. & Eastwood, M. Balanced mechanical forces and microtubule contribution to fibroblast contraction. J Cell Physiol 169, 439-47 (1996).
44. Esue, O., Carson, A. A., Tseng, Y. & Wirtz, D. A direct interaction between actin and vimentin filaments mediated by the tail domain of vimentin. J Biol Chem (2006).
45. Wang, N. & Stamenovic, D. Mechanics of vimentin intermediate filaments. J Muscle Res Cell Motil 23, 535-40 (2002).
46. Eckes, B. et al. Impaired wound healing in embryonic and adult mice lacking vimentin. J Cell Sci 113 ( Pt 13), 2455-62 (2000).
47. Stitt, A. W. et al. Advanced glycation end products (AGEs) co-localize with AGE receptors in the retinal vasculature of diabetic and of AGE-infused rats. Am J Pathol 150, 523-31 (1997).
48. Nieminen, M. et al. Vimentin function in lymphocyte adhesion and transcellular migration. Nat Cell Biol 8, 156-62 (2006).
49. Nishio, K., Inoue, A., Qiao, S., Kondo, H. & Mimura, A. Senescence and cytoskeleton: overproduction of vimentin induces senescent-like morphology in human fibroblasts. Histochem Cell Biol 116, 321-7 (2001).
50. Ikeda, K. et al. N (epsilon)-(carboxymethyl)lysine protein adduct is a major immunological epitope in proteins modified with advanced glycation end products of the Maillard reaction. Biochemistry 35, 8075-83 (1996).

## Claims

1. Use of N-acetylhydroxyproline for retarding skin ageing in a subject.

2. Use according to claim 1, wherein N-acetylhydroxyproline is applied topically to skin.

3. A cosmetic or pharmaceutical composition which comprises N-acetylhydroxyproline in combination with one or more substances which are selected from one or more of groups (a) to (e):
(a) starch-based particles which are substantially non-swellable with water and have an average particle size of from about 1 µm to about 100 µm;
(b) emulsifiers comprising at least one carbohydrate moiety;
(c) thickeners selected from one or more of acryloyldimethyltaurate/vinylpyrrolidone copolymers, acrylic acid/vinylpyrrolidone crosspolymers, polyacrylates and polyesters;
(d) UV filters selected from one or more of 2,4-bis(((2-ethylhexyloxy)-2-hydroxyl)-phenyl)-6-(4-methoxyphenyl)-(1,3,5)-triazine, terephthalidene dicamphor sulfonic acid, 2,2'-methylene-bis-6-(2H-benzotriazol-2-yl)-4-(tetramethylbutyl)-1,1,3,3-phenol, diethylamino hydroxybenzoyl hexyl benzoate and 2,2'-(1,4-phenylene)-bis(1H-benzimidazole-4,6-disulfonic acid, monosodium salt)
(e) skin benefit agents selected from one or more of rutin and derivatives thereof, coenzyme Q10, creatine, L-carnitine, genistein, daidzein, licochalcone A, folic acid and vitamin B3 (niacinamide).
